(19) Europäisches Patentamt
European Patent Office
Office européen des brevets

(11) EP 1 082 963 A1

(12) **EUROPEAN PATENT APPLICATION**
published in accordance with Art. 158(3) EPC

(43) Date of publication:
**14.03.2001 Bulletin 2001/11**

(21) Application number: **99921167.5**

(22) Date of filing: **19.05.1999**

(51) Int. Cl.[7]: **A61K 31/725**, C08B 37/08, A61K 45/00
// (A61K31/725, 31:40)

(86) International application number:
**PCT/JP99/02600**

(87) International publication number:
**WO 99/59603 (25.11.1999 Gazette 1999/47)**

(84) Designated Contracting States:
**AT BE CH CY DE DK ES FI FR GB GR IE IT LI LU MC NL PT SE**

(30) Priority: **20.05.1998 JP 13832998**
**07.08.1998 JP 22418798**
**22.02.1999 JP 4306499**

(71) Applicant:
**CHUGAI SEIYAKU KABUSHIKI KAISHA**
**Tokyo, 115-0051 (JP)**

(72) Inventors:
- **TAMURA, Tatsuya**
  **Gotenba-shi, Shizuoka 412-8513 (JP)**
- **OKAMACHI, Akira**
  **Gotenba-shi, Shizuoka 412-8513 (JP)**

(74) Representative: **HOFFMANN - EITLE**
**Patent- und Rechtsanwälte**
**Arabellastrasse 4**
**81925 München (DE)**

# (54) REMEDIES FOR JOINT DISEASES BOUND TO HYALURONIC ACID

(57) The object of the present invention is to provide a conjugate of a therapeutic agent for joint diseases and hyaluronic acid, a hyaluronic acid derivative or a salt thereof which can retain the therapeutic agent for joint diseases in joint cavities. According to the present invention, there are provided a conjugate of at least one therapeutic agent for joint diseases and hyaluronic acid, a hyaluronic acid derivative or a salt thereof; a method for preparing the above described conjugate which conjugate comprises binding a site of the therapeutic agent for joint diseases (for example, a matrix protease inhibitor) which does not affect the activity of a therapeutic agent to a carboxyl group, a hydroxyl group or a functional group at the reducing end of hyaluronic acid, a hyaluronic acid derivative or a salt thereof by direct chemical reaction or via a spacer; and a pharmaceutical composition containing the above described conjugate.

EP 1 082 963 A1

## Description

FIELD OF THE INVENTION

The present invention relates to a conjugate of hyaluronic acid, a derivative thereof or a salt thereof and a therapeutic agent for joint diseases. More specifically, the present invention relates to a conjugate obtained by chemically binding hyaluronic acid, a derivative or a salt thereof to a therapeutic agent for joint diseases which agent is effective for treating osteoarthritis, rheumatoid arthritis and the like, a method for preparing the conjugate, and a pharmaceutical composition containing the conjugate.

BACKGROUND OF THE INVENTION

Articular cartilage is composed of about 70% of water, chondrocytes and a cartilage matrix. The major components constituting the articular matrix are collagen and proteoglycan; the proteoglycan having good water retention characteristics is contained in the network of collagen having a reticulated structure. The articular matrix is rich in viscoelasticity and has an important role in reducing the stimulus and load imposed on the cartilage in order to maintain the normal morphology and function of the articular cartilage.

Osteoarthritis (OA) and rheumatoid arthritis (RA) are representative of the diseases accompanied by the destruction of the cartilage matrix. It is thought that the destruction of the matrix in these diseases is triggered by mechanical stresses with aging in the case of OA and by excess proliferation of the surface layer cells of the synovial membrane, pannus formation and inflammatory cell infiltration in the case of RA, and both phenomena are caused through the induction of proteases. Since the degradation of articular cartilage is progressed in the extracellular region at a neutral pH, it is said that a matrix metalloprotease (hereinafter referred to as "MMP" or "MMPs" when used as the general term) whose optimal pH is in the neutral range plays a leading role in the degradation.

Up to now, as to humans, 16 types of proteases which belong to the MMP family have been reported; 4 types of endogenous proteins which bind to the proteases and inhibit their activities have been found and named tissue metalloprotease inhibitors (hereinafter referred to as "TIMP" or TIMPs" when used as the general term). MMPs exhibit various functions such as genesis, angiogenesis, estrous cycle, bone remodeling and tissue repair in physiological conditions. In order to appropriately exhibit these functions, each step of the production, the activation and the interaction with the substrate of MMPs is strictly controlled by TIMPs and other endogenous protease inhibitors. In other words, it is thought that the destruction of the matrix in diseased conditions is caused by some failures in the controlling mechanism, resulting in excessive production and activation of MMPs.

Therefore, drugs inhibiting MMPs are extremely promising as the drugs inhibiting the destruction of cartilage matrix in joint diseases such as OA and RA. Many drugs inhibiting MMPs have been reported; among them, hydroxamic acids are most noted because of their strong inhibiting activity and high specificity to MMPs. Hydroxamic acids capable of inhibiting MMP even by oral administration have been found and some of which have been already applied to clinical trials on cancer patients and arthritis patients.

However, MMP inhibitors of this type more or less show inhibiting activity against all types of MMPs and inhibit even the MMPs taking part in physiological functions. In fact, in the clinical trials in progress of hydroxamic acids on patients of cancer, side effects such as transient skeletal muscle pains and tendinitis have been reported. Recently, improved products having heightened specificity to certain MMPs are under development, however no MMPs involved in diseased conditions alone have been found yet. Furthermore, since novel MMPs are found one after another, there still remains a possibility that some physiological actions of MMPs are inhibited when an MMP inhibitor is systemically administered.

The local administration of a hydroxamic acid into a joint cavity may first be proposed as an effective way to solve the above-described problems. However, frequent administration is required in order to maintain the local concentration of the hydroxamic acid; for the patients of OA and RA who unavoidably receive administration of the hydroxamic acid over a long time period, such frequent administration is very disadvantageous. The use of a so-called drug delivery system which restrictively localizes the hydroxamic acid at the target site may be proposed as alternative method. However, no methods for restrictively localizing or retaining the administered hydroxamic acid within the morbid joint have been established.

As mentioned above, although hydroxamic acids have excellent pharmacological properties, there still remain problems to be solved before they can be clinically applied as a therapeutic agent for chronic diseases such as OA and RA.

The intraarticular injection of hyaluronic acid (hereinafter also referred to as "HA") and crosslinked product thereof (hereinafter also referred to as "HA formulation" as the general term for hyaluronic acid and its crosslinked product) currently finds extensive clinical application to joint diseases, especially OA and scapulohumeral periarthritis.

Hyaluronic acid (HA) is an endogenous polysaccharide constituted by repeating units of N-acetylglucosamine and glucronic acid and, as the major component constituting the synovial fluid, it plays an important role in retaining the visco-elasticity of the synovial fluid, the load absorption function and the lubrication function. Furthermore, in the cartilage matrix, HA binds to cartilage proteoglycan to form a polymer called aglycan and plays a central role in maintaining the water retaining ability and viscoelasticity of the cartilage matrix.

It is said that as a lubricant and also by enhancing the HA production in joints and the like, HA formulations ease the disorder of joint functions, although they do not inhibit MMPs. HA has a strong affinity to the exracellular matrix, since HA is inherently a constituent of the extracellular matrix, and in addition, HA has high viscoelasticity in itself; accordingly, HA is characteristically localized within the joint cavity for a long time period after it is injected into the joint cavity. In fact, in an experiment using $^{14}C$ labeled HA, it has been reported that the $^{14}C$ labeled HA as administered into a rabbit knee joint cavity is distributed to synovial fluid, synovial membrane tissue, the surface layer of articular cartilage and the like and it takes at least three days before the HA disappears from those tissues. Furthermore, it is said that HA does not undergo degradation in the synovial fluid and is partially degraded in the synovial membrane tissue and the articular cartilage but most of the HA slowly transfers into blood through the synovial membrane and decomposes into lower molecular substances in the liver.

Therefore, if a drug bound to HA is administered to a living body, it is expected that the drug is retained together with the HA at a specific site for a long period of time and the duration of the drug action at the specific site is remarkably prolonged as compared to the case of administrating the drug alone. Furthermore, it is expected that by such an effect the dosage of the drug and the frequency of drug administration are remarkably reduced as compared to the conventional administrating method, resulting in greatly relieved side effects.

As HA-drug conjugates there are known an interferon/hyaluronic acid conjugate as described in Japanese Patent Publication (Kokai) No. Hei 5-85942/1993, a hyaluronic acid/anticancer agent conjugate as described in WO92/06714 Publication, a hyaluronic acid/corticosteroid conjugate as described in Japanese Patent Publication (Kokai) No. Sho 62-64802/1987, a hyaluronic acid/antibiotic conjugate as described in Japanese Patent No. 2701865 and the like.

However, in most of those cases, the effect of the drug is exhibited only after the drug is liberated from HA, typically by decomposition of HA into lower molecular substances or by hydrolysis of the bond between HA and the drug, and taken up by the target cells or tissues.

DISCLOSURE OF THE INVENTION

One object of the present invention is to provide a conjugate of a therapeutic agent for joint diseases (for example, matrix metalloprotease inhibitors, particularly matrix metalloprotease inhibitors capable of retaining a hydroxamic acid in a joint cavity, other non-steroidal anti-inflammatory drugs, cyclooxgenase-2 inhibitors, disease-modifying anti-rheumatic agents and steroids) and hyaluronic acid, a derivative thereof or a salt thereof.

Another object of the present invention is to provide a method for preparing the conjugate.

Still another object of the present invention is to provide a pharmaceutical composition containing the conjugate.

The present inventors have noted that there is a case (Moore W. M. & Spilburg C. A., Biochemistry 25, 5189-5195 (1986)) in which a hydroxamic acid having MMP inhibiting activity has been proved to maintain the bindability to MMPs even when it is coupled to agarose which is one of artificial polysaccharides, and that all MMPs that have ever been discovered are exhibiting their enzymatic functions extracellularly or on the surface layer of cells. As the result of strenuous investigations made to solve the above described problems, the present inventors have found that a conjugate (e.g., a covalent conjugate of hydroxiamic acid and HA formulation) prepared by allowing a therapeutic agent for joint diseases (e.g., an MMP inhibitor or another non-steroidal anti-inflammatory agent, a cyclooxynage-2 inhibitor, a disease-modifying antirheumatic agent or a steroid) to chemically bind to HA, an HA derivative or an HA salt exhibits MMP inhibition even in the conjugate form. The present invention has been achieved on the basis of this finding.

In addition, the present inventors have found that similar to HA formulations, the conjugate of a therapeutic agent for joint diseases and HA, a derivative or a salt thereof remained in a joint cavity for a long period of time after being administered into the joint cavity, thereby reducing systemic side effects accompanying the MMP inhibitor and maintaining the medical effect of HA as the therapeutic agent for joint diseases; in other words, the present inventors have found that since the synergistic medicinal efficacy of HA and the therapeutic agent for joint diseases can be expected to manifest in the local site, the conjugate can be used as a pharmaceutical composition having improved biological utility. The present invention has been achieved on the basis of this finding.

According to a first aspect of the present invention, there is provided a conjugate of (1) at least one therapeutic agent for joint diseases and (2) hyaluronic acid, a hyaluronic acid derivative or a salt thereof.

In one mode of the present invention, the bond between the therapeutic agent for joint diseases and hyaluronic acid, the hyaluronic acid derivative or the salt thereof is a covalent bond.

In another mode of the present invention, the therapeutic agent for joint diseases is a matrix metalloprotease

inhibitor.

In another mode of the present invention, the matrix metalloprotease inhibitor binds to hyaluronic acid, the hyaluronic acid derivative or the salt thereof via a spacer.

In the conjugate of the present invention, the weight ratio of the matrix metalloprotease inhibitor to the entire conjugate is not particularly limited but is preferably 0.01 to 50%, more preferably 0.1 to 10%.

In the conjugate of the present invention, the matrix metalloprotease inhibitor is preferably a hydroxamic acid residue.

The matrix metalloprotease inhibitor is particularly preferably a hydroxamic acid residue represented by the general formula (1),

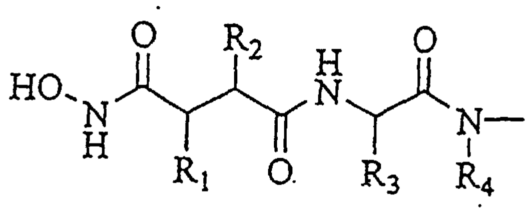

(1)

wherein

$R_1$ is a hydrogen atom, a hydroxyl group or a straight-chain or branched-chain alkyl group having 1 to 8 carbon atoms;
$R_2$ is a straight-chain or branched-chain alkyl group having 1 to 8 carbon atoms;
$R_3$ is a straight-chain or branched-chain alkyl group having 1 to 8 carbon atoms which may be substituted with a cycloalkyl group, an aryl group or a heterocyclic group; and
$R_4$ is a hydrogen atom or a straight-chain or branched-chain alkyl group having 1 to 4 carbon atoms.

In the conjugate of the present invention, if there exists a spacer between the matrix metalloprotease inhibitor and the hyaluronic acid component, the spacer is particularly preferably represented by the general formula (2),

$$-R_5-R_6-R_7-R_8- \quad (2)$$

wherein

$R_5$ is a straight-chain or branched-chain alkyl group having 1 to 8 carbon atoms;
$R_6$ is a methylene group or an imino group, both of which may be substituted with a straight-chain or branched-chain alkyl group having 1 to 4 carbon atoms, or an oxygen atom;
$R_7$ is a straight-chain or branched-chain alkylene group having 1 to 10 carbon atoms into which one to three oxygen atoms may be inserted; and
$R_8$ is an oxygen atom, a sulfur atom or $NR_9$ wherein
$R_9$ is a hydrogen atom or a straight-chain or branched-chain alkyl group having 1 to 4 carbon atoms.

In the conjugate of the present invention, preferred examples of the conjugate of a matrix protease inhibitor and a spacer are represented by the general formula (3),

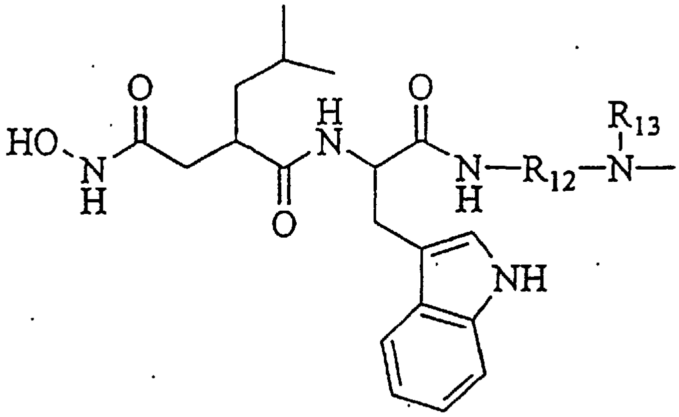

(3)

wherein

$R_{12}$ is a straight-chain or branched-chain alkylene group having 2 to 23 carbon atoms into which one imino group and/or one to four oxygen atoms may be inserted; and

$R_{13}$ is a hydrogen atom or a straight-chain or branched-chain alkyl group having 1 to 4 carbon atoms.

Furthermore, when the conjugate is administered to a living body, the matrix metalloprotease inhibitor, which is in the form of the conjugate with hyaluronic acid, a hyaluronic acid derivative or a salt thereof, inhibits matrix metalloproteases.

According to a second aspect of the present invention, there is provided a method for preparing the conjugate of the present invention which comprises binding a site of a therapeutic agent for joint diseases that does not affect the activity of the agent to a carboxylic group, a hydroxyl group or a functional group at the reducing end of hyaluronic acid, a hyaluronic acid derivative or a salt thereof by direct chemical reaction or via a spacer. Thus, in the above described preparation method, a carboxylic group, a hydroxyl group or a functional group at the reducing end of hyaluronic acid, a hyaluronic acid derivative or a salt thereof is bound to a site of a therapeutic agent for joint diseases (for example, a matrix metalloprotease inhibitor) that does not affect the activity of the therapeutic agent either by direct chemical chemical reaction or via a spacer; binding via a spacer is performed in expectation of the possibility that at the time of binding reaction, the spacer is allowed to react with HA, a hyaluronic acid derivative or a salt thereof without being sterically affected by the therapeutic agent (e.g., MMP inhibitor) by virtue of the space to be created between the therapeutic agent and the reaction point at the distal end of the spacer and/or that in a conjugate, by virtue of the space to be created between the therapeutic agent for joint diseases (e.g., MMP inhibitor) and HA, a hyaluronic acid derivative or a salt thereof, MMP will come sufficiently close to the therapeutic agent without being sterically effected by the HA, HA derivative or salts thereof that the MMP inhibiting activity of the therapeutic agent is maintained even in the conjugate form.

According to a third aspect of the present invention, there is provided a pharmaceutical composition comprising the conjugate of the present invention.

The pharmaceutical composition of the present invention is particularly a therapeutic agent for joint diseases, more specifically, a therapeutic agent for osteoarthritis, rheumatoid arthritis or scapulo-humeral periarthritis.

<u>BRIEF DESCRIPTION OF THE DRAWINGS</u>

Fig. 1 includes graphs showing the inhibiting activity of a conjugate of the present invention against various MMPs (the upper graph shows the inhibiting activity against collagenase-1 and the lower graph shows the inhibiting activity against stromelysin-1).

Fig. 2 includes graphs showing the inhibiting activity of the conjugate against various MMPs (the upper graph shows the inhibiting activity against gelatinase A and the lower graph shows the inhibiting activity against gelatinase B).

Fig. 3 is a graph showing the inhibiting activity of a conjugate of the present invention against the destruction of collagen films.

Fig. 4 is a graph showing the bound-stability of a conjugate of the present invention (the stability of conjugate 5 at 37°C in physiological saline).

Fig. 5 is a graph showing the bond-stability of a conjugate of the present invention (the permeability of conjugate 4 to a semipermeable membrane).

Fig. 6 is a graph showing the retainability of the conjugate in rat joint cavities.

Fig. 7 is a graph showing the retainability of the conjugate in rat joint cavities.

Fig. 8 includes graphs showing the inhibiting activity of a conjugate of the present invention against various MMPs (the upper graph shows the inhibiting activity against collagenase-1 and the lower graph shows the inhibiting activity against stromelysin-1).

Fig. 9 includes graphs showing the inhibiting activity of the conjugate against various MMPs (the upper graph shows the inhibiting activity against gelatinase A and the lower graph shows the inhibiting activity against gelatinase B).

Fig. 10 is a graph showing the inhibiting activity of the conjugate on the destruction of articular cartilage collagen. In Fig. 10, mark * indicates a significant difference from the group containing interleukin 1 and plasminogen [$p<0.05$, Dunnett multiple comparison test, mean value ± standard error ($n=4$)].

PREFERRED MODE FOR CARRYING OUT THE INVENTION

In the present invention, therapeutic agents for joint diseases include, for example,

(1) non-steroidal anti-inflammatory agents including, for example, salicylic acid based non-steroidal anti-inflammatory agents (such as sasapyrine, aspirin, diflunisal, and salicylamide); fenamic acid based non-steroidal anti-inflammatory agents (such as fulfenamic acid, aluminum fulfenamate, mefenamic acid, floctafenine, and tolfenamic acid); arylacetic acid based non-steroidal anti-inflammatory agents (such as dichlorofenac sodium salt, tolmetin sodium salt, sulindac, fenbufen, indomethacin, indomethacin farnesyl, acemetacin, proglumetacin maleate, amfenac sodium salt, nabumetone, mofezolac, etdolac, and alclofenac); propionic acid based non-steroidal anti-inflammatory agents (such as ibuprofen, flurbiprofen, ketoprofen, naproxen, pranoprofen, fenoprofen calcium salt. tiaprofenic acid, oxaprozin, loxoprofen sodium salt, aluminoprofen, zaltoprofen, and tiaprofenic acid); pyrazolone based non-steroidal anti-inflammatory agents (such as ketophenylbutazone); oxicam based non-steroidal anti-inflammatory agents (such as piroxicam, tenoxicam and ampiloxicam; basic non-steroidal anti-inflammatory agents (such as tialamide hydrochloride, tinoridine hydrochloride, benzydamine hydrochloride, epirizole and emorfazone);

(2) cyclooxygenase-2 inhibitors (such as celecoxib, a product of Searle, MK-966 a product of Merck, and JTE 522, a product of Japan Tobacco Inc.);

(3) antirheumatic agents including, for example, penicillamine, disodium lobenzarit, auranofin, bucillamine, actarit, salazosulfapyridine, sodium aurothiomalate, chloroquine, TNF α receptors [for example, Enbrel (registered trademark, a product of American Home Poducts), mizoribine, cyclosporin, methotrexate, leflunomide (a product of Hoechst Marion Roussel), azathioprine, FK-506 (a product of Fujisawa Pharmaceutical Co., Ltd.), VX-497 (a product of Vertex), TAK-603 (a product of Takeda Chemical Industries, Ltd.), anti-TFN α antibodies [for example, infliximab (a product of Centocor) and D2E7 (a product of Knoll Chemische Fabriken AG)], anti-interleukin 6 receptor antibodies [for example, MRA (a product of Chugai Pharmaceutical Co., Ltd.)], T-614 (a product of Toyama Chemical Co., Ltd.), KE-298 (a product of Taisho Pharmaceutical Co., Ltd.), mynophenolate mofetil (a product of Roche), thalidomide (a product of Celgen), anti-CD4 antibodies, interleukin 1 acceptor antagonists, anti-CD52 antibodies, p38MAP kinase inhibitors, ICE inhibitors, and TACE inhibitors;

(4) steroids (such as cortisone acetate, hydrocortisone, prednisolone, methylprednisolone, triamcinolone, triamoinolone acetonide, dexamethasone, dexamethasone palmitate, betamethasone, paramethasone acetate, halopredone acetate, prednisolone farnesylate and tetracosactide acetate);

(5) local anesthetics including, for example, procaine hydrochloride, tetracaine hydrochloride, and lidocaine hydrochloride; and

(6) cartilage protective agents including, e.g., matrix metalloprotease inhibitor.

Among them, a matrix metalloprotease inhibitor is preferred.

In the present invention, a matrix metalloprotease (MMP) means all substances that can inhibit the activity of any matrix metalloprotease derived from any living body (preferably mammals, particularly preferably humans) by, for example, binding thereto.

More specifically, matrix metalloprotease inhibitors mean: compounds or proteins (including polypeptides) which inhibit the enzymatic activity of MMPs by binding to zinc, which is the active center of the MMPs, via a functional group such as a carboxylic acid, a phosphoric acid, a thiol and a hyroxamic acid; and those which inhibit expression of the enzymatic activity of MMPs or proteolytic enzymes having both disintegrin and MMP-like domains in their molecules

[for example, TNFα converting enzyme or a group of proteases belonging to a disintegrin/metalloprotease family (ADAM)]. The activity of these MMP inhibitors can be measured, for example, as the inhibiting activity against the degradation by MMPs of labeled substrates (described in Cawston, T.E. & Barrett, A.J Anal. Biochem., 99, 340-345 (1979) and Baici, A et al. Anal. Biochem., 108, 230-232 (1980)) and synthetic substrates (Masui, Y et al. Biochm. Med., 17, 215-221(1997)); more conveniently, it can similarly be measured by using commercially available MMP activity measuring kits developed on the basis of the above methods. The activity of these MMP inhibitors can also be measured as the inhibiting activity against the production and activation of MMPs and TNFα converting enzymes in an experimental system cited in Gavrilovic, J et al. (Cell. Biol. Int. Reports, 9, 1097-1107 (1985) and Br. J. Pharmcol., 100, 631-635 (1990), in which system cells cultured on a film of a substrate such as collagen are stimulated by a cytokine so that the activity of MMPs thus produced and activated is measured using the liberation of the decomposed substrate into the culture medium as an indicator or in another experimental system (DiMartino et al. (Inflam. Res., 46, 211-215 (1997)) in which peripheral leukocytes are stimulated by a lipopolysaccharide and the like so that the thus induced liberation of TNFα from the surface layer of cell membranes is evaluated as the activity of the TNFα converting enzyme. The above described MMP inhibitors are characterized in that they exhibit 50% or more inhibition at any concentration of 10 mg/ml or less in at least one of those measuring systems. MMPs inhibitors also include those inhibitors whose structural formulae are chemically modified, provided that such inhibitors exhibit an inhibiting activity of at least 45% of inhibition at any concentration of 10 mg/ml or less.

Non-limiting specific examples of MMP inhibitors include tetracycline compounds (such as tetracycline, doxycycline, minocycline and chemical modifications of tetracycline (for example, CMT 1 to 4, products of Collagenex)), TIMPs, and hydroxamic acids, and from the standpoints of the strength of MMP inhibiting activity and high specificity to MMPs, hydroxamic acids are preferred.

Examples of such MMP inhibitors are described in, for example, Japanese Patent Publication (Kokai) No. Hei 9-80825/1997, Japanese Patent No. 2736285 and Drug Discovery Today, 1, 16-26 (1996).

A hydroxamic acid means a compound having an N-hydroxyamide group, and non-limiting specific examples of hydroxamic acid include, for example, AG-3340 (a product of Agouron), CDP-845 (a product of Zeneca), CGS-27023A (a product of Novartis), D5410 (a product of Chiro Science), L758354 (a product of Merck), CH-138 (a product of Chiro Science), Marimastat (registered trademark, a product of British Biotec), Galardin (registered trademark, a product of Glycomed), Ro31-9790 (a product of Roche), Bay 12-9566 (Bayer), and RS 130830 (Roche Bioscience). Further, non-limiting specific examples of the hydroxamic acid residues in the conjugates of the present invention include, for example, hydroxamic acid residues represented by the general formula (1),

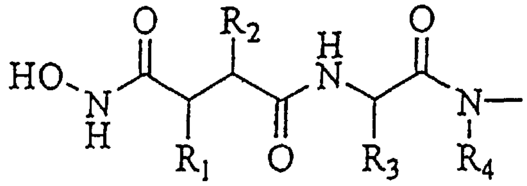

(1)

wherein

- $R_1$ is a hydrogen atom, a hydroxyl group or a straight-chain or branched-chain alkyl group having 1 to 8 carbon atoms;
- $R_2$ is a straight-chain or branched-chain alkyl group having 1 to 8 carbon atoms;
- $R_3$ is a straight-chain or branched-chain alkyl group having 1 to 8 carbon atoms which may be substituted with a cycloalkyl group, an aryl group or a heterocyclic group; and
- $R_4$ is a hydrogen atom or a straight-chain or branched-chain alkyl group having 1 to 4 carbon atoms.

In the definition of the hydroxamic acid residues of the MMP inhibitors represented by the general formula (1), non-limiting specific examples of $R_1$ include a hydrogen atom, a hydroxyl group, a methyl group, an ethyl group, an n-propyl group, an isopropyl group, an n-butyl group, a sec-butyl group, an isobutyl group, a tert-butyl group, an n-pentyl group, an n-hexyl group, an n-heptyl group, and an n-octyl group, and a hydrogen atom is preferred.

Non-limiting specific examples of $R_2$ include a methyl group, an ethyl group, an n-propyl group, an isopropyl group, an n-butyl group, a sec-butyl group, an isobutyl group, a tert-butyl group, an n-pentyl group, an n-hexyl group, an n-heptyl group, and an n-octyl group, and an isobutyl group is preferred.

Non-limiting specific examples of the alkyl group component in the straight-chain or branched-chain alkyl group having 1 to 8 carbon atoms which may be substituted with a cycloalkyl group, an aryl group or a heterocyclic group in $R_3$ include a methyl group, an ethyl group, an n-propyl group, an isopropyl group, an n-butyl group, a sec-butyl

group, an isobutyl group, a tert-butyl group, an n-pentyl group, an n-hexyl group, an n-heptyl group, and an n-octyl group, and preferred are a methyl group, an isobutyl group and a tert-butyl group.

Further, non-limiting specific examples of the cycloalkyl group, the aryl group or the heterocyclic group which may be present on the above described alkyl groups include cycloalkyl groups having 3 to 10 carbon atoms, preferably 5 to 7 carbon atoms (such as a cyclopentyl group, a cyclohexyl group, and a cycloheptyl group); aryl groups having 6 to 20 carbon atoms, preferably 6 to 14 carbon atoms (such as a phenyl group, a p-hydroxyphenyl group, and a naphthyl group) which may have a substituent such as a hydroxyl group and a methoxy group; and saturated or unsaturated heterocyclic rings (such as a pyridyl group, a quinolyl group, and a 3-indolyl group, preferably a 3-indolyl group) having 5-20 atoms, preferably 5 to 10 atoms, particularly preferably of 5, 6, 9 or 10 atoms and containing one or more hetero atoms which may be the same or different preferably 1 to 3 hetero atoms, particularly preferably one hetero atom, as selected from among a nitrogen atom, a sulfur atom and an oxygen atom.

To give typical examples, $R_3$ is preferably a straight chain alkyl group having 1 to 5 carbon atoms which may be substituted with an aryl group or a heterocyclic group and above all, particularly preferred are a benzyl group, a p-hydroxybenzyl group, and a 3-indolylmethyl group, and a 3-indolylmethyl group is the most preferred.

Non-limiting specific examples of $R_4$ include a hydrogen atom, a methyl group, an ethyl group, an n-propyl group, an isopropyl group, an n-butyl group, a sec-butyl group, an isobutyl group, and a tert-butyl group, and preferred is a hydrogen atom.

The hydroxamic acid residues represented by the general formula (1) contain at least one asymmetric center and as to each asymmetric center, its absolute configuration may be the R-configuration or the S-configuration in the present invention.

The weight ratio of the matrix metalloprotease inhibitor is preferably 0.01 to 50%, particularly preferably 0.1 to 10% based on the whole conjugate.

Further, in the conjugate of at least one therapeutic agent for joint diseases and hyaluric acid, a hyaluronic acid derivative or a salt thereof, MMP inhibitors as a preferred therapeutic agent for joint diseases might change their structures in the course of the synthesis of the conjugate or after the synthesis; even if their structures change, such MMP inhibitors are included in the present invention if they have the inhibiting activity described in the present specification (at least one of MMP inhibition, inhibition of collagen destruction, and inhibition of TNF$\alpha$ liberation).

In the present invention, "hyaluronic acid (HA)" means disaccharide polymers which have a weight average molecular weight of 100,000 to 10,000,000 and which are composed of glucuronic acid and N-acetylglucosamine, and a mixture of those polymers. From the standpoint of the strength in viscoelasticity, hyaluronic acid having a weight average molecular weight of 700,000 to 10,000,000 is preferred and hyaluronic acid having a weight average molecular weight of 1,000,000 to 10,000,000 is particularly preferred.

In the present invention, "a hyaluronic acid derivative " means all substances that are derived from hyaluronic acid and which have a hyaluronic acid skeleton. Non-limiting specific examples of the hyaluronic acid derivative include:

(1) hyaluronic acid derivatives in which glucuronic acid and/or N-acrylglucosamine which are the sugar component has a reducing end;

(2) acetylated hyaluronic acid in which at least one hydroxyl group in hyaluronic acid is acetylated;

(3) derivatives of disaccharide polymers which have a weight average molecular weight of 100,000 to 10,000,000, which are composed of glucuronic acid and N-acetylglucosamine and whole molecular weight is further increased by croslinking with formaldehyde (an example of such derivatives is Synvisc (registered trademark, a product of Biomatrix)); and

(4) derivatives obtained by allowing hyaluronic acid or the hyaluronic acid derivatives as described above in the present specification to bind, via a spacer or without a spacer, to at least one pharmaceutically effective component such as an anticancer agent (for example, an alkylating agent, a metabolic antagonist, and an alkaloid), an immunosuppressive agent, an anti-inflammatory agent (such as a steroid, a non-steroidal anti-inflammatory agent), an antirheumatic agent or an antibacterial agent (such as a $\beta$-lactam antibiotic, an aminoglycoside antibiotic, a macrolide antibiotic, a tetracycline antibiotic, a new quinolone antibiotic, a polypeptide antibiotic, and a sulfa agent).

Non-limiting specific examples of salts of hyaluronic acid and the hyaluronic acid derivatives include a sodium salt, a potassium salt, a magnesium salt, a calcium salt and an aluminum salt.

Although there is no limitation in the origin of HA, HA originated from bacteria such as *Actinomyces*, humans, pigs, and chicks can be used.

Non-limiting specific examples of hyaluronic acid and salts thereof include, for example, Suvenyl (registered trademark, Japan Roussel), Artz (registered trademark, Kaken Pharmaceutical Co., Ltd.), Opegan (registered trademark, Santen Pharmaceutical Co., Ltd.), Hyalgan (registered trademark, Fidia), Orthobisk (registered trademark, Anika Therapeutics), and Healon (registered trademark, Pharmacia & Upjohn). Further, HA and the salts thereof as described

in the catalogs of various reagent makers such as Wako Pure Chemical Industries, Ltd. can also be included.

In the conjugate of the present invention, a therapeutic agent for joint diseases (for example, a matrix metalloprotease inhibitor) is bound to hyaluronic acid, a hyaluronic acid derivative or a salt thereof via a spacer(s) or without any spacer. As the mode of binding between the therapeutic agent for joint diseases (for example, a matrix metalloprotease inhibitor) and hyaluronic acid, a hyaluronic acid derivative or a salt thereof, bonds such as an amide bond and an ether bond can be used in the absence of a spacer; or they are allowed to bind via a spacer(s). Preferably, the therapeutic agent for joint diseases (for example, a matrix metalloprotease inhibitor) binds to hyaluronic acid, the hyaluronic acid derivative or the salt thereof via at least one spacer.

When the therapeutic agent for joint diseases (for example, a matrix metalloprotease inhibitor) binds to hyaluronic acid, the hyaluronic acid derivative or the salt thereof without a spacer, they bind to each other at sites that do not adversely affect their activities. In the preferred mode of the present invention in which the therapeutic agent for joint diseases (for example, a matrix metalloprotease inhibitor) binds to hyaluronic acid, the hyaluronic acid derivative or the salt thereof via a spacer(s), the spacer(s) and the therapeutic agent for joint diseases or the spacer(s) and hyaluronic acid, the hyaluronic acid derivative or the salt thereof bind to each other at sites that do not adversely affect the activities of the therapeutic agent for joint diseases or hyaluronic acid, the hyaluronic acid derivative or the salt thereof.

As to the therapeutic agent for joint diseases, such sites that do not adversely affect their activities include, for example, an amino group, a carboxyl group, a hydroxyl group, and a thiol group. In a preferred mode of the present invention in which an MMP inhibitor which is a therapeutic agent for joint diseases is the hydroxamic acid residue represented by the general formula (1), such sites include a primary or secondary amino group positioned at the terminal end of the residue. As to hyaluronic acid, the hyaluronic acid derivative or the salt thereof, such sites include, for example, a hydroxyl group and a carboxyl group, preferably a carboxyl group.

The type of the bond between the therapeutic agent for joint diseases (for example, an MMP inhibitor) and the HA, the HA derivative or the salt thereof, the type of the bond between the spacer and the therapeutic agent for joint diseases (for example, an MMP inhibitor), and the type of the bond between the spacer and the HA, HA derivative or salt thereof are not particularly limited; for example, an amide bond, an ether bond, an ester bond, and a sulfide bond can be used.

The therapeutic agent for joint diseases binding to HA, an HA derivative or a salt thereof is not necessarily limited to one type, and two or more different types of therapeutic agents for joint diseases may be used. Further, one conjugate may have both a binding site interrupted by a spacer(s) and a binding site not interrupted by a spacer(s). Furthermore, spacers present in one conjugate are not necessarily the same.

The type of the spacers is not limited unless the activities of the therapeutic agent for joint diseases (for example, an MMP inhibitor) and the HA, the HA derivative or the salt thereof are materially affected; non-limiting specific examples of the spacers include a spacer represented by the general formula (2),

$$-R_5-R_6-R_7-R_8- \qquad (2)$$

wherein

$R_5$ is a straight-chain or branched-chain alkylene group having 1 to 8 carbon atoms;
$R_6$ is a methylene group or an imino group which may be substituted with a straight-chain or branched-chain alkyl group having 1 to 4 carbon atoms or an oxygen atom;
$R_7$ is a straight-chain or branched-chain alkylene group having 1 to 10 carbon atoms into which one to three oxygen atoms may be inserted; and
$R_8$ is an oxygen atom, a sulfur atom or $NR_9$ (wherein
$R_9$ is a hydrogen atom or a straight-chain or branched-chain alkyl group having 1 to 4 carbon atoms.

The spacer represented by the above described general formula (2) binds to a therapeutic agent for joint diseases (for example, an MMP inhibitor) at the $R_5$-end thereof and binds to HA, an HA derivative or a salt thereof at the $R_8$-end thereof.

In the definition of the spacer represented by the above described general formula (2), non-limiting specific examples of $R_5$ include a methylene group, an ethane-1,2-diyl group, a propane-1,3-diyl, a butane-1,4-diyl group, a pentane-1,5-diyl group, a hexane-1,6-diyl group, a heptane-1,7-diyl group, an octane-1,8-diyl group, a 2-methylpentane-1,3-diyl group, 2-methylbutane-1,4-diyl group, a 3-methylbutane-1,4-diyl group, a 3-methylpentane-1,5-diyl group, a 3-ethylpentane-1,5-diyl group, a 3-methylhexane-1,6-diyl group, a 4-methylhexane-1,6-diyl group, and a 4-methylheptane-1,7-diyl group, and preferred are an ethane-1,2-diyl group, a propan-1,3-diyl group, and a butane-1,4-diyl group.

Examples of the straight-chain or branched-chain alkyl group having 1 to 3 carbon atoms in the methylene group or imino group which may be substituted with a straight-chain or branched-chain alkyl group having 1 to 4 carbon

atoms include a methyl group, an ethyl group, an n-propyl group, an isopropyl group, an n-butyl group, a sec-butyl group, and a tert-butyl group.

To give typical examples, $R_6$ is preferably a methylene group which may be substituted with a straight-chain or branched-chain alkyl group having 1 to 3 carbon atoms or an oxygen atom, and particularly preferred is a methylene group or an oxygen atom.

Non-limiting specific examples of $R_7$ include a methylene group, an ethane-1,2-diyl group, a propane-1,3-diyl group, a butane-1,4-diyl group, a pentane-1,5-diyl group, a hexane-1,6-diyl group, a heptane-1,7-diyl group, an octane-1,8-diyl group, a nonane-1,9-diyl group, an octane-1,10-diyl group, a 2-methylpentane-1,3-diyl group, a 2-methylbutane-1,4-diyl group, a 3-methylbutane-1,4-diyl group, a 3-methylpentane-1,5-diyl group, a 3-ethylpentane-1,5-diyl group, a 3-methylhexane-1,6-diyl group, a 4-methylhexane-1,6-diyl group, a 4-methylheptane-1,7-diyl group, a 1-oxapropane-1,3-diyl group, a 2-oxabutane-1,4-diyl group, a 3-oxapentane-1,5-diyl group, a 2-oxahexane-1,6-diyl group, a 3-oxahexane-1,6-diyl group, a 1,4-oxahexane-1,6-diyl group, a 3-oxaheptane-1,7-diyl group, a 2,5-dioxahepane-1,7-diyl group, a 4-oxaoctane-1,8-diyl group, a 2,6-dioxaoctane-1,8-diyl group, a 3,6-dioxanonane-1,9-diyl group, a 3,6-dioxa-4-methylnonane-1,9-diyl group, a 3,6-dioxa-5-ethylnonane-1,9-diyl group, and 1,4,7-trioxaoctane-1,10-diyl group, and preferred are an ethane-1,2-diyl group, a propane-1,3-diyl group, a butane-1,4-diyl group, a 3,6-dioxanonane-1,9-diyl group, etc.

Non-limiting specific examples of $R_8$ include an oxygen atom, a sulfur atom, an imino group, a methylimino group, an ethylimino group, an n-propylimino group, an isopropylimino group, an n-butylimino group, a sec-butylimino group, an isobutylimino group, and a tert-butylimino group, and preferred is an imino group or a methylimino group, and particularly preferred is an imino group.

Preferred specific examples of the spacer include $-(CH_2)_4$-NH-, $-(CH_2)_5$-NH-, $-(CH_2)_6$-NH-, $-(CH_2)_7$-NH-, $-(CH_2)_8$-NH-, $-(CH_2)_9$-NH-, $-(CH_2)_{10}$-NH-, $-(CH_2)_{11}$-NH-, $-(CH_2)_{12}$-NH-, $-(CH_2)_2$-O-$(CH_2)_2$-NH-, $-(CH_2)_3$-O-$(CH_2)_3$-NH-, $-(CH_2)_4$-O-$(CH_2)_4$-NH-, and $-(CH_2)_3$-O-$(CH_2)_2$-O-$(CH_2)_2$-O-$(CH_2)_3$-NH-.

Furthermore, in the conjugate in which a therapeutic agent for joint diseases (for example, a matrix metalloprotease inhibitor) and hyaluronic acid, a hyaluronic acid derivative or a salt thereof are bound to each other via at least one spacer, preferred non-limiting specific examples of the conjugate of a therapeutic agent for joint diseases (for example, a matrix metalloprotease inhibitor) to the spacer include conjugates represented by the general formula (3),

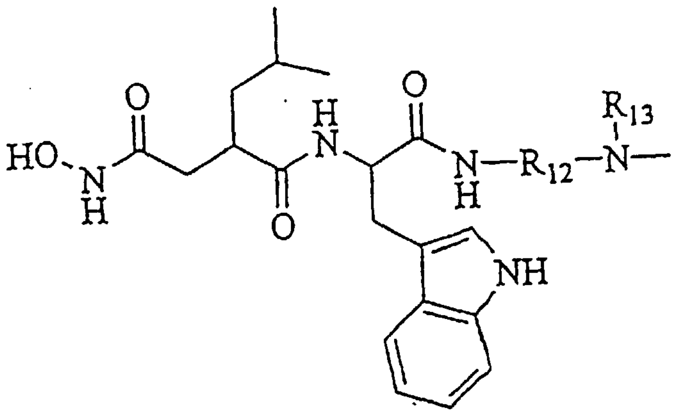

(3)

wherein

$R_{12}$ is a straight-chain or branched-chain alkylene group having 2 to 23 carbon atoms into which one imino group and/or one to four oxygen atoms may be inserted; and
$R_{13}$ is a hydrogen atom or a straight-chain or branched-chain alkyl group having 1 to 4 carbon atoms.

The hydroxamic acid residue moiety in the conjugates represented by the general formula (3) is the same as the preferred example of the MMP inhibitor.

Further, non-limiting specific examples of $R_{12}$ include an ethane-1,2-diyl group, a propane-1,3-diyl group, a butane-1,4-diyl group, a pentane-1,5-diyl group, a hexane-1,6-diyl group, a heptane-1,7-diyl group, an octane-1,8-diyl group, a nonane-1,9-diyl group, a decane-1,10-diyl group, an undecane-1,11-diyl group, a dodecane-1,12-diyl group, a 2-methylpentane-1,3-diyl group, a 2-methylbutan-1,4-diyl group, a 3-methyl-butane-1,4-diyl group, a 3-methylpentane-1,5-diyl group, a 3-ethylpentane-1,5-diyl group, a 3-methylhexane-1,6-diyl group, a 4-methylhexane-1,6-diyl group, a 4-methylheptane-1,7-diyl group, $-(CH_2)_2$-O-$(CH_2)_2$-, $-(CH_2)_3$-O-$(CH_2)_3$-, $-(CH_2)_4$-O-$(CH_2)_4$-, and $-(CH_2)_3$-O-$(CH_2)_2$-O-$(CH_2)_2$-O-$(CH_2)_3$-, and preferred are a butane-1,4-diyl group, a pentane-1,5-diyl group, a hexane-1,6-diyl group, a heptane-1,7-diyl group, an octane-1,8-diyl group, a nonane-1,9-diyl group, a decane-1,10-diyl group, an undecane-1,11-diyl group, a dodecane-1,12-diyl group, $-(CH_2)_2-O-(CH_2)_2-$, $-(CH_2)_3-O-(CH_2)_3-$, $-(CH_2)_4-O-(CH_2)_4-$, and $-(CH_2)_3-O-(CH_2)_2-O-(CH_2)_2-O-(CH_2)_3-$. Non-limiting specific examples of $R_{13}$ include a hydrogen atom, a methyl group, an ethyl group, an n-propyl group, an isopropyl group, an n-butyl group, a sec-butyl group, and a tert-butyl group, and preferred are a hydrogen atom and a methyl group, and particularly preferred is a hydrogen atom.

The spacer represented by the general formula (2) and the conjugate represented by the general formula (3) sometimes have an asymmetric carbon atom(s) in the molecule so that they include stereoisomers having an absolute configuration which is an R-configuration or an S-configuration; each of such stereoisomers or structural units (for the spacer or the conjugate) consisting of those stereoisomers in any proportions are included in the present invention.

Methods for preparing the conjugate of the present invention include, for example, binding by chemical reaction a site (for example, an amino group, a carboxyl group, a hydroxyl group, a thiol group or the like) which does not affect the activity of a therapeutic agent for joint diseases (for example, an MMP inhibitor) to a carboxyl group, a hydroxyl group or an aldehyde group originating from the reducing end of HA, an HA derivative or a salt thereof. This reaction can be carried out by known techniques (as described in "Shinseikagaku Jikken Koza (A New Course in Experimental Biochemistry)", Vol.1, Proteins I" (Tokyo Kagakudojin), "Tanpaku Koso no Kiso Jikken Hou (Basic Experimental Methods for Proteins and Enzymes)" (Nankodo) and the like).

Specific examples are as follows:

(1) a method for activating a carboxyl group in a therapeutic agent for joint diseases (for example, an MMP inhibitor) or HA, an HA derivative or a salt thereof with the use of a dehydrative condensation agent to form an amide bond, an ester bond or a thioester bond;

(2) a method for activating a hydroxyl group in a therapeutic agent for joint diseases (for example, an MMP inhibitor) with the use of cyanogen bromide and then binding the activated group to an amino group in HA, an HA derivative or a salt thereof, and a method for activating a hydroxyl group in HA, an HA derivative or a salt thereof with the use of cyanogen bromide and then binding the activated group to an amino group in a therapeutic agent for joint diseases (for example, an MMP inhibitor);

(3) a method for activating a hydroxyl group in a therapeutic agent for joint diseases or HA, an HA derivative or a salt thereof with the use of a halohydrin such as epichlorohydrin or a diepoxide such as 1,4-butanediol diglycidyl ether or a sulfonyl chloride such as tosyl chloride and tresyl chloride to form an ether bond, an imino bond or a sulfide bond; and

(4) a method for reducing the reducing end in HA, an HA derivative or a salt thereof to form a primary hydroxyl group, oxidizing the hydroxyl group to form an aldehyde group, and subjecting the resulting aldehyde to reductive alkylation with an amine in a therapeutic agent for joint diseases (for example, an MMP inhibitor).

If desired, two or more of the above described methods (1) to (4) may be combined.

In the method for activating a carboxyl group in a therapeutic agent for joint diseases or HA, an HA derivative or a salt thereof with the use of a dehydrative condensation agent to form an amide bond, an ester bond or a thioester bond, condensation agents which are used in the general organic synthesis can be employed, and preferably carbodiimides, phosphoniums, uroniums and the like are used. Carbodiimides include, for example, non-water soluble carbodiimides such as diisoprpyl carbodiimide and dicyclohexyl carbodiimde, and water soluble carbodiimides such as 1-ethyl-3-(3-dimethylaminopropyl)carbodiimide; phosphoniums include, for example, benzotriazol-1-yloxytris(dimethylamino)phosphonium hexafluorophosphate and 7-azabenzotriazol-1-yloxytris(dimethylamino)phosphonium hexafluorophosphate; and uroniums include, for example, O-benzotriazol-1-yl-N,N,N,N-tetramethyluronium hexafluorophosphate and O-7-azabenzotriazol-1-yl-N,N,N,N-tetramethyluronium hexafluorophosphate.

Further, a reaction accelerating additive may be added to those condensation agents. Examples of such an additive include N-hydroxysuccinimide, N-hydroxy-5-norbornene-2,3-dicarboximide, p-nitrophenol, pentafluorophenol, 1-hydroxybenzotriazole, and 1-hydroxy-7-azabenzotriazole.

Condensation by a water soluble carbodiimide is a non-limiting specific example of the method for activating carboxyl group in a therapeutic agent for joint diseases (for example, an MMP inhibitor) or HA, an HA derivative or a salt thereof with the use of a dehydrative condensation agent to form an amide bond, an ester bond or a thioester bond. In this method, a carbodiimide is added to a 0.1 to 1% (weight/volume) HA aqueous solution, and subsequently a therapeutic agent for joint diseases (for example, an MMP inhibitor) containing an amino group is added to the resulting solution and reaction is performed at 0°C to 35°C for 1 to 96 hours. During this reaction, an acid such as hydrochloric acid or phosphoric acid can be added to maintain the pH of the reaction solution at 4 to 6.

If the therapeutic agent for joint diseases (for example, an MMP inhibitor) to be used has low water solubility, an aqueous solution containing 1 to 50% of an organic solvent (for example, N,N-dimethylformamide, N-methylpyrrolidone, dioxane, ethanol, pyridine or the like) can be used as the reaction solvent. In this case, the therapeutic agent for joint diseases (for example, an MMP inhibitor) may first be added to the reaction system and the carbodiimide nay be

added after confirming that the therapeutic agent has dissolved.

If desired, a reaction accelerating additive (for example, N-hydroxysuccinimide, N-hydroxy-5-norbornene-2,3-dicarboximide, p-nitrophenol, pentafluorophenol, 1-hydroxybenzotriazole, 1-hydroxy-7-azabenzotriazole or the like) and HA may preliminarily be treated with a dehydrative condensation agent in order to convert a carboxyl group in HA to an active ester, which is isolated and then mixed with a therapeutic agent for joint diseases (for example, an MMP inhibitor) for reaction.

The following are non-limiting specific examples of the method for activating a hydroxyl group in a therapeutic agent for joint diseases (for example, an MMP inhibitor) with the use of cyanogen bromide and then binding it to an amino group in HA, an HA derivative or a salt thereof and the method for activating a hydroxyl group in HA, an HA derivative or a salt thereof with the use of cyanogen bromide and then binding it to an amino group in the therapeutic agent for joint diseases (for example, MMP inhibitor):

To an aqueous solution of HA, an HA derivative or a salt thereof, cyanogen bromide is added and reaction is performed at 0°C to 10°C for 5 to 30 minutes. During the reaction, the pH can be maintained at 10 to 12 with sodium hydroxide or a phosphate buffer solution. Acetonitrile is then added to the reaction mixture to form a precipitate and excess cyanogen bromide is removed; the precipitate is reconstituted into an aqueous solution, mixed with a therapeutic agent for joint diseases (for example, an MMP inhibitor) having an amino group and subjected to reaction at 4°C to 25°C for 1 to 24 hours. During the reaction, the pH of the reaction mixture can be maintained at 8 to 10 with sodium bicarbonate, sodium hydroxide or the like.

The following are non-limiting specific examples of the method for reducing the reducing end of HA, an HA derivative or a salt thereof to form a primary hydroxyl group, oxidizing it to form an aldehyde group and subjecting the resulting aldehyde group to reductive alkylation with an amine in a therapeutic agent for joint diseases (for example, an MMP inhibitor):

Treatment with a reducing agent such as sodium borohydride and subsequent treatment with an oxidizing agent such as sodium periodate produces HA, an HA derivative or a salt thereof having an aldehyde group at the reducing end; to the obtained solution by treating the HA, the HA derivative or the salt thereof, a therapeutic agent for joint diseases (for example, an MMP inhibitor) having an amino group is added; to the resulting mixture, sodium cyanoborohydride is added and reaction is performed at 15°C to 30°C for 1 to 24 hours. During the reaction, the pH of the reaction mixture may be maintained at 4 to 6 by adding acetic acid, hydrochloric acid, phosphoric acid or the like.

In any of these condensation methods, the desired conjugate can be obtained by adding an organic solvent such as ethanol and acetone to the reaction mixture after the reaction to form a precipitate, which is then purified by a suitable means such as alcohol precipitation, gel filtration, dialysis, or ion-exchange chromatography.

If the conjugate of the present invention which comprises a therapeutic agent for joint diseases bound to HA, HA derivative or a salt thereof is to be used as a drug, it is preferably used after being formulated into a pharmaceutical preparation together with a pharmaceutically acceptable diluting agent, stabilizer and the like.

The mode of administration of the drug or pharmaceutical composition is not particularly limited and may be oral or parenteral and may be systemic or local. In general, the pharmaceutical composition of the present invention is preferably administrated parenterally and locally, for example, intraarticularly, intraveneously, intramuscularly or intradermally as injection, or percutaneously as a spraying agent, a topical cream or an ointment.

The dosage of the pharmaceutical composition of the present invention can suitably be selected depending on the condition of the disease, age and, sex of the patient and the like; in the case of using it as injection, the amount of the conjugate (i.e., the effective ingredient) ranges from 0.01 mg/body weight in kg/day to 100 mg/ body weight in /kg/day, preferably from 0.1 mg/body weight in kg /day to 10 mg/body weight in kg /day. The above described daily dosage per day may be administered in several divided portions a day or administered once a day or once in 2 to 28 days.

<u>Example</u>

Example 1: Synthesis of MMP Inhibitor

<u>(a) N-Benzyloxycarbonyl-1,4-diaminobutane</u>

1,4-Diaminobutane (10g, 113 mmol) was dissolved in water/ ethanol(100 ml : 300 ml), and with stirring under cooling with ice a solution of benzyloxycarbonyl chloride (19.35g, 113 mmol) in 1,2-dimethoxyethane (50 ml) was added dropwise over about 30 minutes. After a 2N sodium hydroxide aqueous solution (2 ml) was added, the resulting solution as such was stirred under cooling with ice for three hours and then stirred at 4°C for 15 hours. After most of the solvent was distilled off under reduced pressure, the residue was dissolved in water and acidified with concentrated hydrochloric acid. The resulting solution was washed with chloroform (100 ml x 2) and then the aqueous layer was alkalized with a 2N sodium hydroxide aqueous solution, followed by extraction with chloroform. The resulting organic layer was washed with a saturated sodium chloride aqueous solution, dried over sodium sulfate and then the solvent was distilled

off under reduced pressure to give 11.0 g of an oil. (Yield 44%)

$^1$H-NMR(270 MHz, $CDCl_3$): δ 1.4-1.5(4H, m), 2.7(2H, t), 3.2(2H, t),5.1(2H, s),7.3-7.4(5H, m)
MS: 222 ($M^+$)

(b) N-9-Fluorenylmethyloxycarbonyl-L-tryptophan-N-(4-N-benzyloxycarbonylaminobutyl)amide

With stirring under cooling with ice, 1-ethyl-3-(3-dimethylaminopropyl)carbodiimide hydrochloride salt (EDC)(1.12 g, 5.85 mmol) was added to a solution (20 ml) of N-9-fluorenylmethyloxycabonyltryptophan (2.22 g, 4.5 mmol) and 1-hydroxybenzotriazole (0.90 g, 5.85 mmol) in N,N-dimethylformamide (DMF) and stirred for one hour. To the reaction solution, the above obtained N-benzyloxycarbonyl-1,4-diaminobutane (1g, 4.5 mmol) was added, the resulting mixture as such was stirred under cooling with ice and then stirring was continued at 15°C to 30°C for 15 hours. After most of the solvent had been distilled off under reduced pressure, the residue was dissolved in chloroform (100 ml) and washed with a 0.5N hydrochloric acid aqueous solution (40 ml x 2), a saturated sodium bicarbonate aqueous solution (50 ml) and a saturated sodium chloride aqueous solution (50 ml). The organic layer was dried over anhydrous sodium sulfate and then concentrated. The resulting residue was purified by silica gel column chromatography using chloroform/methanol as the eluting solution to obtain 2.1 g of colorless powder. (Yield 74%)

$^1$H-NMR(270 MHz, $CDCl_3$): δ 2.2-3.4(10H, m), 4.2(1H, t), 4.3-4.5(3H,m), 5.1(2H, s), 7.0-8.0(18H, m)

(c) L-Tryptophan-N-(4-N-benzyloxycarbonylaminobutyl)amide

The condensation product (2.1 g) as obtained in (b) was dissolved in DMF (50 ml), and piperidine (3 ml) was added to the solution; the resulting solution was stirred at 15°C - 30°C for 30 minutes. After most of the solvent was distilled off under reduced pressure, the residue was purified by silica gel column chromatography using chloroform/methanol as the eluting solution to obtain 1.0 g of a transparent oil. (Yield 74%)

$^1$H-NMR(270 MHz, $CDCl_3$): δ 1.4(4H,m), 3.0-3.4(6H,m), 3.7(1H,m), 5.1(2H,s), 7.0-7.7(9H,m)
MS:408($M^+$)

(d) [4-(N-Benzyloxyamino)-2-isobutylsuccinyl]-L-tryptophan-N-(4-N-benzyloxycarbonylaminobutyl)amide:(Compound 1a)

L-Tryptophan-N-(4-N-benzyloxycarbonylaminobutyl)amide (1.18 g, 2.9 mmol) was dissolved in DMF (30 ml), and with stirring under cooling with ice, 4-(N-benzyloxyamino)-2-isobutylsuccinic acid (732 mg, 2.6 mmol) as synthesized according to a known method [Japanese Patent Publication (Kokai) No. Hei 6-145148/1994] and EDC (552 mg, 2.9 mmol) were successively added; the reaction temperature was set between the temperature of cooling with ice and that of cooling with water, and stirring was continued for three days. The reaction solution was concentrated under reduced pressure and diluted with chloroform; the chloroform layer was successively washed with 0.1N hydrochloric acid, water, a saturated sodium bicarbonate aqueous solution and a saturated sodium chloride aqueous solution, and dried over sodium sulfate. After filtration, the residue and the aqueous layer were re-extracted with ethyl acetate; the ethyl acetate layer and the chloroform layer were combined and concentrated under reduced pressure. The obtained crude product was subjected to silica gel chromatography purification (WAKO, C-200, eluting solvents: chloroform and a 1 : 1 mixture of chloroform and acetone); the resulting fractions were collected and concentrated under reduced pressure and dried to obtain 1.20 g (68%) of the title compound 1a.

MS: 670 ($M+H^+$)

(e) [4-(N-hydroxyamino)-2(R)-isobutylsuccinyl]-L-tryptophan-N-(4-N-aminobutyl)amide:(Compound 2)

[4-(N-hydroxyamino)-2(S)-isobutylsuccinyl]-L-tryptophan-N-(4-N-aminobutyl)amide:(Compound 3)

[4-(N-Benzyloxyamino)-2-isobutylsuccinyl]-L-tryptophan-N-(4-N-benzyloxycarbonylaminobutyl)amide (Compound 1a) (1.20 g, 1.8 mmol) was dissolved in 50 ml of methanol and catalytically reduced with 140 mg of 10% Pd/C under an atmospheric pressure of hydrogen for 16 hours. The reaction solution was filtered with celite and then concetrated under reduced pressure. The obtained crude product was subjected to reverse phase HPLC (column: YMC-Pack, ODS, 250 mm x 20 mm I.D., eluting solvent: a 0.1% trifluoroactic acid (TFA)-containing water/acetonitrile system, flow rate: 10 ml/min) and respective diastereomers were recovered and purified and freeze-dried to obtain 283

mg of a TFA salt of the title compound 2 (peak at the hydrophilic side) and 493 mg of a TFA salt of the title compound 3 (peak at the hydrophobic side), respectively. Compound 2:

$^1$H-NMR(270 MHz, $CD_3OD$):0.70(3H,d,J=6Hz), 0.77(3H,d,J=6Hz), 1.02-1.53(7H,m), 2.12(1H,dd,J=14,5Hz), 2.29(1H,dd,J=14,9Hz), 2.59-2.68(1H,m), 2.80-2.85(2H,m), 3.10-3.36(4H,m), 4.49-4.58(1H,m), 6.96-7.09(3H,m), 7.30(1H,d,J=8Hz), 7.57(1H,d,J=8Hz), 7.95-8.04(2H,m)
MS:446($M+H^+$)

Compound 3:

$^1$H-NMR(270 MHz,$CD_3OD$):0.51(3H,d,J=6Hz), 0.56(3H,d,J=6Hz), 0.63-0.92(2H,m),1.11-1.21(1H,m), 1.56-1.58(4H,m), 2.02(1H,dd,J=15,2Hz),2.31(1H,dd,J=15,11Hz),2.48-2.60(1H,m), 2.86-3.45(6H,m), 4.64-4.72(1H,m), 6.91-7.04(3H,m), 7.27(1H,d,J=8Hz), 7.54(1H,d,J=8Hz), 7.97-8.08(2H,m)
MS:446($M+H^+$)

(f) N-Benzyloxycarbonyl-1,8-dimaminooctane

In the same manner as in the synthesis of N-bnezyloxcarbonyl-1,4-diaminobutane in (a), 1,8-diaminooctane instead of 1,4-diaminobutane as the starting material was treated to obtain 6.8 g of the title compound as an oil (yield 58%).

$^1$H-NMR(270MHz,$CDCl_3$):δ 1.3(8H,s),1.4-1.5(4H,m), 2.7(2H,t,J=7Hz), 3.2(2H,m), 5.1(2H,s), 7.3-7.4(5H,m)
MS:278($M^+$)

(g) N-9-Fluorenylmethyloxycarbonyl-L-tryptophan-N-(8-N-benzyloxycarbonylaminooctyl)amide

With stirring under cooling with ice, EDC (3.90 g, 20.5 mmol) was added to a solution of N-9-fluorenylmethyloxycarbony-L-tryptophan (7.8 g, 15.8 mmol) and 1-hydroxybenzotriazole (3.15 g, 20.0 mmol) in DMF (100 ml) and stirred for one hour. To the reaction solution, the N-benzyloxycarbonyl-1,8-diaminooctane (4.4 g, 15.8 mmol) as obtained above was added, the resulting solution as such was stirred under cooling with ice and then stirring was continued at 15°C - 30°C for 15 hours. Most of the solvent was distilled off under reduced pressure; the residue was dissolved in chloroform (200 ml) and washed with a 0.5N hydrochloric acid aqueous solution (50 ml x 3), a saturated sodium bicarbonate aqueous solution (100 ml) and a saturated sodium chloride aqueous solution (50 ml). The organic layer was dried over anhydrous sodium sulfate and then concentrated and used as such in the next reaction.

(h) L-Tryptophan-N-(8-N-benzyloxycarbonylaminooctyl)amide

The condensation product as obtained in (g) was dissolved in DMF (150 ml) and then piperidine (10 ml) was added to the solution; the resulting solution was stirred at 15°C - 30°C for 30 minutes. After most of the solvent was distilled off under reduced pressure, the residue was purified by silica gel column chromatography using chloroform/methanol as the eluting solution to obtain 6.1 g of a yellow oil. (Yield from N-benzyloxycarbonyl-1,8-diaminooctane: 74%)

$^1$H-NMR(270 MHz, $CDCl_3$):δ 1.2-1.6(12H,m),2.9-3.4(6H,m), 3.7(1H,m), 5.1(2H,s), 7.0-7.7(9H,m)
MS:465($M^+$)

(i) [4-(N-Benzyloxyamino)-2-isobutylsuccinyl]-L-tryptophan-N-(8-N-benzyloxcarbonylaminooctyl)amide: (Compound 4)

In the same manner as in the synthesis of compound 1a, L-tryptophan-N-(8-N-benzyloxcarbonylaminooctyl)amide (2.07 g, 4.5 mmol) instead of L-tryptophan-N-(4-N-benzyloxycarbonylaminobutyl)amide as the starting material was treated to obtain 2.5 g of the title compound (yield: 85%). The reaction medium used was 30 ml of DMF and the reaction time employed was 2 days. Further, the reaction residue concentrated under reduced pressure was diluted with ethyl acetate without re-extraction. For silica gel chromatography purification, chloroform and a 1 : 1 mixture of chloroform and acetone were used as eluting solvents. The obtained title compound as such was used in the next reaction.

(j) [4-(N-Hydroxyamino)-2(R)-isobutylsuccinyl]-L-tryptophan-N-(8-N-aminooctyl)amide:(Compound 5)

[4-(N-Hydroxyamino)-2(S)-isobutylsuccinyl]-L-tryptophan-N-(8-N-aminooctyl)amide:(Compound 6)

In the same manner as in the syntheses of compound 3 and compound 4, 1.7 g (yield 100%) of a diastereomer mixture (compound 7) of the title compound 5 and the title compound 6 were obtained by using [4-(N-benzyloxyamino)-2-isobutylsuccinyl]-L-tryptophan-N-(8-N-benzyloxycarbonylaminooctyl)amide (Compound 4) (2.5 g, 3.4 mmol) instead of [4-(N-benzyloxyamino)-2-isobutylsuccinyl]-L-tryptophan-N-(4-N-benzyloxycarbonylaminobutyl)amide (1) as the starting material. A portion (360 mg) of the diastereomer mixture was subjected to reverse phase HPLC, so that the respective diastereomers were recovered and purified; subsequent freeze-drying gave 151 mg of a TFA salt of the title compound 5 (peak at the hydrophilic side) and 147 mg of a TFA salt of the title compound 6 (peak at the hydrophobic side). Compound 5:

$^1$H-NMR(270MHz,DMSO-$d_6$):0.74(3H,d,J=6Hz), 0.79(3H,d,J=6Hz), 0.97-1.59(15H,m), 1.91(1H,dd,J=14,8Hz), 2.03(1H,dd,J=14,7Hz), 2.62-2.83(3H,m), 2.89-3.12(4H,m), 4.40-4.48(1H,m), 6.95(1H,dd,J=7,7Hz), 7.04(1H,dd,J=7,7Hz), 7.11(1H,d,J=2Hz), 7.30(1H,d,J=8Hz), 7.54(1H,d,J=8Hz), 7.58-7.81(4H,m), 8.01(1H,d,J=8Hz), 8.73(1H,s), 10.38(1H,s), 10.78(1H,s)
MS:502(M+H$^+$)

Compound 6:

$^1$H-NMR(270MHz,DMSO-$d_6$):0.55(3H,d,J=5Hz), 0.66(3H,d,J=5Hz), 0.75-1.59(15H,m), 1.94(1H,dd,J=15,5Hz), 2.14(1H,dd,J=15,9Hz), 2.57-3.38(7H,m), 4.32-4.44(1H,m), 6.95(1H,dd,J=7,7Hz), 7.04(1H,dd,J=7,7Hz), 7.10(1H,brs), 7.30(1H,d,J=8Hz), 7.53(1H,d,J=8Hz), 7.65(3H,brs), 7.90(1H,t,J=6Hz), 8.19(1H,d,J=8Hz), 8.73(1H,brs), 10.45(1H,s), 10.78(1H,s)
MS:502(M+H$^+$)

(k) N-Benzyloxycarbonyl-4,7,10-trioxa-1,13-tridecanediamine

In the same manner as in the synthesis of N-benzyloxycarbonyl-1,4-diaminobutane in (a), the title compound was obtained as 5.0 g of an oil by using 4,7,10-trioxa-1,13-tridecanediamine instead of N-benzyloxycarbonyl-1,4-diaminobutane as the starting material. (Yield 39%)

$^1$H-NMR(270MHz,$CDCl_3$):δ 1.6-1.7(4H,m), 2.8(2H,t,J=6.7Hz), 3.3(2H,m), 3.5-3.6(12H,m), 5.1(2H,s), 5.6(1H,brs), 7.3-7.4(5H,m)
MS:354(M$^+$)

(l) N-9-Fluorenylmethyloxycarbonyl-L-tryptophan-N-(13-N-benzyloxycarbonylamino-4,7,10-trioxa-tridecanyl)amide

In the same manner as in the synthesis of N-9-fluorenylmethyloxycarbony-L-tryptophan-N-(4-N-benzyloxycarbonylaminobutyl)amide in (b), the title compound was obtained as 8.0 g of an oil by using N-benzyloxycarbonyl-4,7,10-trioxa-1,13-tridecanediamine instead of N-benzyloxycarbonyl-1,4-diaminobutane as the starting material. (Yield 39%)

$^1$H-NMR(270MHz,$CDCl_3$):δ 1.42-1.59(2H,m), 1.64-1.75(2H,m), 3.09-3.32(10H,m), 3.42-3.60(8H,m), 4.20(1H,t,J=6.8Hz), 4.31-4.50(3H,m), 5.06(2H,s), 5.24(1H,brs), 5.70(1H,brs), 6.08(1H,brs),6.99(1H,s), 7.07-7.19(2H,m), 7.27-7.42(10H,m), 7.54-7.58(2H,m), 7.66(1H,d,J=7.3Hz), 7.76(2H,d,J=7.6Hz), 8.89(1H,brs)
MS:785.6(M+Na$^+$)

(m) L-Tryptophan-N-(13-N-benzyloxycarbonylamino-4,7,10-trioxa-tridecanyl)amide

In the same manner as in the synthesis of L-tryptophan-N-(4-N-benzyloxycarbonylaminobutyl)amide in (c), the title compound was obtained as 4.2 g of an oil by using N-9-fluorenylmethyloxycarbonyl-L-tryptophan-N-(13-N-benzyloxycarbonylamino-4,7,10-trioxa-tridecanyl)amide instead of N-9-fluorenylmethyloxycarbonyl-L-tryptophan-N-(4-N-benzyloxycarbonylaminobutyl)amide as the starting material. (Yield 78%)

$^1$H-NMR(270MHz,$CDCl_3$):δ 1.64-1.77(4H,m), 2.95-3.04(1H,m), 3.23-3.36(7H,m), 3.45-3.69(11H,m), 5.08(2H,s), 5.34(1H,brs), 7.05-7.21(3H,m), 7.26-7.38(6H,m), 7.66(1H,d,J=7.6Hz), 8.51(1H,brs)

MS:541($M^+$)

(n) [4-(N-benzyloxyamino)-(2R)-isobutylsuccinyl]-L-tryptophan-N-(13-N-benzyloxycarbonylamino-4,7,10-trioxa-tridecanyl)amide:(Compound 8)

The title compound 8 was obtained as 1.15 g (yield 72%) of a colorless amorphous substance in the same manner as in the synthesis of compound 1a, except that L-tryptophan-N-(13-N-benzyloxycarbonylamino-4,7,10-tiroxa-tridecanyl)amide (1.30 g, 2.4 mmol) instead of L-tryptophan-N-(4-N-benzyloxycarbonylaminobutyl)amide and 4-(N-benzyloxyamino)-(2R)-isobutylsuccinic acid (0.56 g, 2.0 mmol) synthesized by a known method [Japanese Patent Publication (Kokai) No. Hei 6-145148/1994] were used as the starting materials. The reaction solvent used was 20 ml of DMF, the reaction temperature employed was 15°C - 30°C, and the reaction time used was 6 hours. Further, the reaction residue concentrated under reduced pressure was diluted with ethyl acetate; the chloroform layer was successively washed with a potassium hydrogensulfate aqueous solution, water, a saturated potassium carbonate aqueous solution, and a saturated sodium chloride aqueous solution, and dried over magnesium sulfate. For silica gel chromatography purification, ethyl acetate and a 9 : 1 mixture of dichloromethane and methanol were used as eluting solvents.

$^1$H-NMR(270MHz,DMSO-$d_6$):δ 0.74(3H,d,J=5.9Hz), 0.80(3H,d,J=6.5Hz), 0.93-1.05(1H,m), 1.29-1.41(2H,m), 1.51-1.58(2H,m),1.60-1.67(2H,m), 1.94(1H,dd,J=14.0,7.3Hz), 2.08(1H,dd,J=14.3,7.3Hz), 2.65 2.78(1H,m), 2.92-3.14(6H,m), 3.26(2H,t,J=6.5Hz), 3.38-3.48(12H,m), 4.47(1H,dt,J=7.8,6.7Hz), 4.76(2H,s), 5.00(2H,s), 6.94(1H,dd,J=7.6,7.2Hz), 7.04(1H,dd,J=8.1,7.2Hz), 7.12(1H,s), 7.22(1H,t,J=5.7Hz), 7.29-7.34(11H,m), 7.55(1H,d,J=7.6Hz), 7.79(1H,t,J=5.4Hz), 8.05(1H,d,J=7.8Hz), 10.78(1H,s), 11.01(1H,s)

(o) [4-(N-hydroxyamino)-(2R)-isobutylsuccinyl]-L-tryptophan-N-(13-N-amino-4,7,10-trioxa-tridecanyl)amide:

(Compound 9)

[4-(N-benzyloxyamino)-(2R)-isobutylsuccinyl]-L-tryptophan-N-(13-N-benzyloxycarbonylamino-4,7,10-trioxa-tridecanyl)amide (Compound 8) (1.90 g, 2.4 mmol) was dissolved in 200 ml of methanol, added with 200 mg of sodium bicarbonate, and catalytically reduced with 200 mg of 10% Pd/C at an atmospheric pressure of hydrogen for three hours. The reaction solution was filtered with celite and then concentrated under reduced pressure to obtain the title compound 9 as 1.50 g (yield 99%) of a colorless amorphous substance.

$^1$H-NMR(270 MHz, $CD_3OD$):δ 0.84(3H,d,J=5.9Hz), 0.89(3H,d,J=6.2Hz), 1.17(1H,ddd,J=11.9,7.6,5.1Hz), 1.38-1.54(2H,m), 1.56-1.65(2H,m),1.71-1.81(2H,m), 2.15(1H,dd,J=14.9,7.4Hz), 2.28(1H,dd,J=14.3,7.4Hz), 2.78(1H,t,J=6.8Hz), 2.80(1H,brs), 3.09-3.32(6H,m), 3.44-3.49(2H,m), 3.52-3.65(8H,m), 4.62(1H,t,J=7.3Hz), 7.04(1H,dd,J=7.6,7.0Hz), 7.12(1H,dd,J=8.0,7.0Hz), 7.15(1H,s), 7.37(1H,d,J=8.0Hz), 7.65(1H,d,J=7.6Hz)
MS:578(M+$H^+$)

Example 2: Conjugate Synthesis Example 1

To 70 mg of an MMP inhibitor (compound 2), 0.49 ml of N-methylpyrrolidone and 0.01 ml of pyridine were added to dissolve the inhibitor; the pH of the solution was adjusted to 4.7 with 0.045 ml of 1M hydrochloric acid and water and its whole volume was adjusted to 1 ml. To the resulting solution, 5 mg of sodium hyaluronate was added to form a uniform solution. After reconfirming that the pH was 4.7, the reaction solution was added with 1.0 mg of EDC under cooling with ice and stirred for 30 minutes, and further stirred at 15°C - 30°C for 15 hours.
To the reaction solution, 1 ml of 0.1M sodium bicarbonate and 6 ml of ethanol were added to form a precipitate which was then purified by repeating the alcohol precipitation method three times (the method comprising the steps of dissolving the precipitate in 1 ml of a 0.2M sodium chloride aqueous solution, effecting precipitation with 3 ml of ethanol and centrifuging the precipitate), thus producing 4.3 mg of a conjugate ("conjugate 1").
The bonding ratio calculated from the UV absorption at 279 nm derived from an indole ring was 0.84% by weight. This means that 0.76% of the carboxyl group reacted.

Example 3: Conjugate Synthesis Example 2

To 70 mg of an MMP inhibitor (compound 3), 0.49 ml of N-methylpyrrolidone and 0.01 ml of pyridine were added to dissolve the inhibitor; the pH of the solution was adjusted to 4.7 with 0.05 ml of 1M hydrochloric acid and water and its whole volume was adjusted to 1 ml. To the resulting solution, 5 mg of sodium hyaluronate was added to form a uniform solution. After reconfirming that the pH was 4.7, the reaction solution was added with 10 mg of EDC under cooling with ice and stirred for 30 minutes, and further stirred at 15°C - 30°C for 20 hours.

To the reaction solution, 1 ml of 0.1M sodium bicarbonate and 6 ml of ethanol were added to form a precipitate which was then purified by repeating the alcohol precipitation method three times (the method comprising the steps of dissolving the precipitate in 1 ml of a 0.2M sodium chloride aqueous solution, effecting precipitation with 3 ml of ethanol and centrifuging the precipitate), thus producing 3.5 mg of a conjugate ("conjugate 2").

The bonding ratio calculated from the UV absorption at 279 nm derived from an indole ring was 1.1% by weight. This means that 1.0% of the carboxyl group reacted.

Example 4: Conjugate Synthesis Example 3

To 77 mg of an MMP inhibitor (compound 7) 0.603 ml of N-methylpyrrolidone and 0.012 ml of pyridine were added to dissolve the inhibitor; the pH of the solution was adjusted to 4.7 with 0.105 ml of 1M hydrochloric acid and water and its whole volume was adjusted to 1.23 ml. To the resulting solution, 6.2 mg of sodium hyaluronate was added to form a uniform solution. After reconfirming that the pH was 4.7, the reaction solution was added with 24 mg of EDC under cooling with ice and stirred at 4°C for 3 days.

To the reaction solution, 0.123 ml of 1M NaOH and 0.5 ml of ethanol were added and stirred for 30 minutes under cooling with ice and then added with 3 ml of ethanol to form a precipitate which was then purified by repeating the alcohol precipitation method three times (the method comprising the steps of dissolving the precipitate in 1 ml of a 0.2M sodium chloride aqueous solution, effecting precipitation with 3 ml of ethanol and centrifuging the precipitate) thus producing 6.0 mg of a conjugate ("conjugate 3").

The bonding ratio calculated from the UV absorption at 279 nm derived from an indole ring was 1.7% by weight. This means that 1.4% of the carboxyl group reacted.

Example 5: Conjugate Synthesis Example 4

To 189 mg of an MMP inhibitor (compound 7), 1.47 ml of N-methylpyrrolidone and 0.03 ml of pyridine were added to dissolve the inhbitor; the pH of the solution was adjusted to 4.7 with 0.24 ml of 1M hydrochloric acid and water and its whole volume was adjusted to 3 ml. To the resulting solution, 15 mg of sodium hyaluronate was added to form a uniform solution. After reconfirming that the pH was 4.7, the reaction solution was added with 87 mg of EDC under cooling with ice and stirred at 4°C for 24 hours.

To the reaction solution, 1.5 ml of 0.1M sodium bicarbonate and 1.5 ml of ethanol were added and stirred for 30 minutes under cooling with ice, and subsequently added with 9 ml of ethanol to form a precipitate which was then purified by repeating the alcohol precipitation method three times (the method comprising the steps of dissolving the precipitate in 3 ml of a 0.2M sodium chloride aqueous solution, effecting precipitation with 9 ml of ethanol and centrifuging the precipitate) thus producing 13.9 mg of a conjugate ("conjugate 4").

The bonding ratio calculated from the UV absorption at 279 nm derived from an indole ring was 4.9% by weight. This means that 3.9% of the carboxyl group reacted.

Example 6: Conjugate Synthesis Example 5

By repeating the same procedure using the same starting material and reagents as in Synthesis Example 3, 5.7 mg of "conjugate 5" was obtained.

The bonding ratio calculated from the UV absorption at 279 nm derived from an indole ring had good reproducibility as in Synthesis Example 3 and was 1.7% by weight. This means that 1.4% of the carboxyl group reacted.

Example 7: Conjugate Synthesis Example 6

To 145 mg of an MMP inhibitor (compound 9), 0.89 ml of N-methylpyrrolidone and 0.02 ml of pyridine were added to dissolve the inhibitor; the pH of the solution was adjusted to 4.7 with 0.09 ml of 6M hydrochloric acid and water and its whole volume was adjusted to 1.82 ml. To the resulting solution, 9.1 mg of sodium hyaluronate was added to form a uniform solution. After reconfirming that the pH was 4.7, the reaction solution was added with 35 mg of EDC under cooling with ice and stirred at 4°C for 24 hours.

To the reaction solution, 0.375 ml of 0.1M sodium bicarbonate and 0.375 ml of ethanol were added and stirred for 30 minutes under cooling with ice, and subsequently added with 5 ml of ethanol to form a precipitate which was then purified by repeating the alcohol precipitation method three times (the method comprising the steps of dissolving the precipitate in 2 ml of a 0.2M sodium chloride aqueous solution, effecting precipitation with 6 ml of ethanol and centrifuging the precipitate), thus producing 8.2 mg of a conjugate ("conjugate 6").

The bonding ratio calculated from the UV absorption at 279 nm derived from an indole ring was 1.0% by

weight. This means that 0.70% of the carboxyl group reacted.

Example 8: Conjugate Synthesis Example 7

N-Hydroxy-5-norbornene-2,3-dicarboximide (8.9 mg) was dissolved in water, added with 0.01 ml of pyridine, 0.07 ml of 1M hydrochloric acid and water to adjust the pH to 4.7, and the whole volume was adjusted to 1 ml. To this solution, 5 mg of sodium hyaluronate was added to render it uniform. The resulting solution was added with 9.6 mg of EDC under cooling with ice and stirred for 17 hours at 4°C. Under cooling with ice, the resulting solution was added with a 2% sodium acetate buffer solution (pH 6) (0.5 ml) and subsequently added with 4 ml of acetone to form a precipitate. The precipitate was centrifuged and dried under reduced pressure.

To 86 mg of a TFA salt (compound 10) of an MMP inhibitor (compound 9)[as obtained by suspending the MMP inhibitor (compound 9) in distilled water containing 0.1% TFA and freeze-drying the resulting suspension], 0.49 ml of N-methylpyrrolidone and 0.01 ml of pyridine were added to dissolve the TFA salt; the pH of the solution was adjusted to 8.0 with 0.035 ml of 1M hydrochloric acid and water, and its whole volume was adjusted to 1 ml. This solution was added to the above described precipitate, and the resulting mixture was stirred at 4°C for three days.

To the reaction solution, 0.2 ml of 2M sodium chloride aqueous solution and 3 ml of ethanol were added to form a precipitate which was then centrifuged. This precipitate was added with 1 ml of a 0.2M sodium chloride aqueous solution and 0.06 ml of a 1M sodium hydroxide aqueous solution, stirred for one hour under cooling with ice to solubilize the precipitate, and added with 3 ml of ethanol to form a precipitate which was then centrifuged. This precipitate was again added with 1 ml of a 0.2M sodium chloride aqueous solution and 0.06 ml of a 1M sodium hydroxide aqueous solution, stirred for three hours under cooling with ice to solubilize the precipitate, and added with 3 ml of ethanol to form a precipitate which was then centrifuged. Subsequently, the precipitate was dissolved in 1 ml of a 0.2M sodium chloride aqueous solution, and added with 3 ml of ethanol to form a precipitate which was then centrifuged; the resulting precipitate was suspended in 90% ethanol/water, then centrifuged, subsequently dissolved in water and freeze-dried to obtain 6.0 mg of a conjugate ("conjugate 7").

The bonding ratio calculated from the UV absorption at 279 nm derived from an indole ring was 1.1% by weight. This means that 0.78% of the carboxyl group reacted.

Experiment 1: Matrix Metalloprotease (MMP) Inhibiting Activity

The enzyme inhibiting activities of "conjugate 1", "conjugate 7" and HA against collagenase-1, stromelysin-1, gelatinase A and gelatinase B were measured. The inhibiting activities against collagenase-1 and stromelysin-1 were measured by using a type I collagenase activity measuring kit and a stromelysin-1 measuring kit manufactured by Yagai Co., Ltd., and the inhibition activities against gelatinase A and gelatinase B were measured by using a gelatinase activity measuring kit manufactured by Roche Diagnostics Co., Ltd. Results were expressed by average values (n=2) of enzymatic activity, with the enzymatic activity in the absence of conjugate or HA being taken as 100. As shown in Figs. 1, 2, 8 and 9, "conjugate 1" and "conjugate 7" had inhibiting activity against any one of these four types of enzyme but HA exhibited no inhibiting activity.

From these experimental results it was found that "conjugate 1" and "conjugate 7" have, an MMP inhibiting activity which HA does not possess.

Experiment 2: Effect of Spacer on Matrix Metalloprotease (MMP) Inhibiting Activity

Four types of conjugate ("conjugate 1", "conjugate 3", "conjugate 4", and "conjugate 6") in which the length of the spacer between the MMP inhibitor described in Patent No. 2736285 (N-[2-isobutyl-3-(N'-hydroxycarbonylamido)propanoyl]-L-tryptophan methylamide: compound 1) and HA was changed between C4 and C10 were compared in terms of the inhibiting activity against gelatinase A and gelatinase B. Results were expressed by the conjugate or HA concentration ($IC_{50}$ value) necessary for inhibiting 50% of the enzymatic activity occurring in the absence of any conjugates or HA (see Table 1 below). Although the inhibiting activity against gelatinase A tended to become a little stronger with the increasing spacer length, no large difference in the inhibiting activity was recognized between these four types of conjugate; from these results, it was concluded that as for conjugates 1 - 4 which were prepared by the same synthetic method (of mixing HA with an MMP inhibitor and then adding a condensation agent), the effect of spacer length on the inhibiting activity was small.

Further, when "conjugate 6" was compared with "conjugate 7" which was synthesized by a method in which HA was first converted to an active ester and then mixed with an MMP inhibitor to effect reaction, the gelatinase A inhibiting activity of the latter was about 10 times as large as that of the former although both conjugates used the same spacer and had the inhibitor bound in almost the same amount. This fact suggested that depending on the synthesis method employed, the inhibiting activity of the MMP inhibitor in a bound form might change from that of the MMP inhibitor in an unbound state.

Table 1

| Effect of Spacer on MMP Inhibiting Activity | | | |
|---|---|---|---|
| Conjugate | Spacer | Enzyme Inhibiting Activity ($IC_{50}$, mg/ml) | |
| | | Gelatinase A | Gelatinase B |
| Conjugate 1 | $C_4H_8$-NH- | 1 | 0.03 |
| Conjugate 3 | $C_8H_{16}$-NH- | 0.7 | 0.04 |
| Conjugate 4 | $C_8H_{16}$-NH- | 0.2 | 0.02 |
| Conjugate 6 | $C_{10}H_{20}O_3$-NH- | 0.2 | NT |
| Conjugate 7 | $C_{10}H_{20}O_3$-NH- | 0.02 | 0.01 |

Experiment 3: Inhibiting Activity on Collagen Film Destruction

The inhibiting activity on collagen-film destruction was measured according to the method of Gavriovic, J et al. [Cell. Biol. Int. Reports, 9, 1097-1107 (1985)]. Articular chondrocytes obtained from the knee joints of 3-6 week-old rabbits by treatment with collagenase were suspended in 500 µl of Dulbeco's modified eagle's medium (DMEM) containing 0.2% of lactoalbumin, and 48-well culture plates each precoated with a guinea pig'skin-derived type I collagen film, which was prelabeled with $^{14}C$, were seeded with 500 µl aliquots of the suspension. "Conjugate 3" or HA was cultured in the presence of interleukin 1 (1 ng/ml) and plasmin (100 µg/ml) in a $CO_2$ incubator at 37°C for 72 hours. After completion of the culture, the supernatant of the culture and a digestive juice obtained by treating the remaining collagen film with collagenase were recovered, and the respective radioactivity were measured with a liquid scintillation counter. Results were calculated as the mean value (n=2) of the percent destruction of the destroyed collagen film according to the following formula.

Percent Destruction of Collagen Film (%) = [(Radioactivity in Supernatant of Culture)/(Radioactivity in Supernatant of Culture + Remaining Radioactivity in Collagen Film)] x 100

As shown in Fig. 3, "conjugate 3" inhibited the cellular collagen destruction induced by interleukin 1 and plasmin, however HA exhibited no inhibitory effect.
From these results, it is apparent that the conjugate of HA and an MMP inhibitor has an excellent inhibitory effect on the collagen destruction by articular chondrocytes although it cannot be inhibited by HA.

Experiment 4: Bond-stability 1 of Conjugate

"Conjugate 5" was dissolved in a physiological saline at a concentration of 1 mg/ml (to give pH=6.3), incubated at 37°C, and the change in the conjugate was analyzed by gel filtration chromatography.
The column was TSK gel G4000PW (7.5 mm I.D.× 30 cm, a product of Tosoh Corporation); a 50 mM phosphate buffer solution (pH 6) containing 20% EtOH was used as an eluting solvent; the column temperature was 40°C (L-7300, manufactured by Hitachi Ltd.); the flow rate was 0.7 ml/min (L-7100, manufactured by Hitachi Ltd.); and a diode array detector (L-7450H, manufactured by Hitachi Ltd.) was used for detection.
The peak area of the absorption at 279 nm due to an indole ring at voids upon injecting 40 µl of the solution was traced at 0 day, 2 days, and 5 days but no change was observed (Fig. 4). Further, during these 5 days, no new peaks in the lower-molecular region were observed on the HPLC.
From these results, excellent stability of the bond between HA and MMP inhibitor was shown by "conjugate 5".

Experiment 5: Bond-stability 2 of Conjugate

In a diffusion cell (donor side: 1.5 ml, acceptor side: 8.0 ml) which was divided by a semipermeable membrane (Type HC; Millipore) having a membrane pore diameter of 25 nm and which was filled with an isotonic phosphate

buffer solution (pH 7.4), compound 1, a mixture of compound 1 and HA, and "conjugate 4" were placed in the amounts shown below; the breakthrough from the donor side to the acceptor side was calculated from the intensity of fluorescence at a measuring wavelength of 350 nm and expressed as permeability (Fig. 5). Herein, 100% permeability means the concentration at which the whole volume of the agent diffuses to become uniform in the cell.

(1) 50 nmol of compound 1
(2) a mixture of 50 nmol of compound 1 and 0.5 mg of HA
(3) 0.5 mg of "conjugate 4" (having compound 1 bound in an amount equivalent to 50 nmol)

In the case of compound 1 and the mixture of compound 1 and HA, compound 1 quickly permeated the membrane to diffuse toward the acceptor side, however "conjugate 4" did not permeate until after 8 hours and only 2.8% and 3.6% of "conjugate 4" permeated in 24 hours and 48 hours, respectively.

From this result, excellent stability in the bond between HA and MMP inhibitor was shown by "conjugate 4".

<u>Experiment 6: Intraarticular Retainability</u>

The following agents (1 to 3) were administered into right knee joints of 9-10 week old rats (n=4 to 10) and the animals were sacrificed at time intervals; the joint cavities were washed with a total 0.5 ml of a physiological saline to recover a synovial fluid.

Agent 1: 30 nmol of compound 1
Agent 2: a mixture of 30 nmol of compound 1 and 0.3 mg of HA
Agent 3: 0.3 mg of "conjugate 4" (having compound 1 bound in an amount equivalent to 30 nmol)

By using a kit for measuring gelatinase activity manufactured by Roche Diagnostics, the inhibiting activity of the synovial fluid against gelatinase B was calculated according to the following formula.

Gelatinase B Inhibition Activity (%) = [(Enzymatic Activity in the Absence of Synovial Fluid - Enzymatic Activity in the Presence of Added Synovial Fluid)/Enzymatic Activity in the Absence of Synovial Fluid] x 100

On the basis of the dose/inhibition curves for compound 1 and "conjugate 4" against gelatinase B, the amount of the agent remaining in the synovial fluid was calculated as the amount of compound 1 in the case of the groups administered compound 1 either alone or in admixture with HA and as the amount equivalent to compound 1 bound to "conjugate 4" in the case of the group administered "conjugate 4". The results were shown in terms of mean values. As Fig. 6 shows, in the group administered compound 1 alone and the group administered the mixture of compound 1 and HA, the amount of the agent remaining in the joint decreased to approximately 1/3,000 of the initial dose (the amount of the agent at 0 hour in the Figure) two hours after administration and the amount decreased to 1/300,000 of the initial dose six hours after administration in the group administered compound 1 alone and 17 hours after administration in the group administered the mixture of compound 1 and HA. Meanwhile, in the group administered "conjugate 4", 2/5 of the dosage remained two hours after administration and approximately 1/10 of the dosage remained 17 hours after administration.

Fig. 7 shows the gelatinase B inhibiting activity of the synovial fluid recovered from each of the treated groups immediately after administration (at 0 hour in the Figure), two hours after administration and 17 hours after administration. The results were shown by a mean value ± standard deviation. The gelatinase B inhibiting activity of the synovial fluid from the group administered compound 1 alone and the group administered the mixture of compound 1 and HA decreased to 20% two hours after administration and to less than 5% 17 hours after administration while in the group administered "conjugate 4" about 50% of the gelatinase B inhibiting activity remained 17 hours after administration.

From these results it is apparent that the conjugate of HA and an MMP inhibitor can be used as an extremely superior means for increasing the retainability of the MMP inhibitor in joint cavities. Further, the conjugate of HA and an MMP inhibitor retains the MMP inhibiting activity for a long time period in joint cavities and this suggests the possibility of inhibiting the articular destruction over a long period of time even after a single intraarticular administration of the conjugate. In other words, it has been suggested that the conjugate of the present invention in which an MMP inhibitor is bound to HA has better efficacy and retainability as a therapeutic agent for joint diseases than the MMP inhibitor or HA used alone or in combination.

Experiment 7: Inhibiting Activity on Articular Cartilage Collagen Destruction

The inhibiting activity on articular cartilage collagen destruction was measured according to the method of Saito. S et al. [J. Biochem., 122, 49-54(1997)]. Fragments (about 10 mg) of articular cartilage were prepared from the knee joint of a 7-week old rabbit and cultured in 500 µl of Dulbeco's modified eagle' medium (DMEM) on a 48-well culture plate for 24 hours. After exchanging the culture medium with 500 µl of DMEM containing 0.2% of lactoalbumin, "conjugate 7" or HA was added thereto and the resulting culture medium was cultivated in the presence of interluekin 1 (1 ng/ml) and plasminogen (100 µg/ml) in a $CO_2$ incubator at 37°C for 10 days. After completion of the culture, the supernatant of the culture and a digestive juice obtained by treating the fragment of articular cartilage with papain were recovered, and hydrochloric acid was added to give a final concentration of 6N; the resulting solution was subjected to hydrolysis in an autoclave at 110°C for 2 hours. Each of the samples was solidified to dryness by spraying with $N_2$ gas, and then dissolved in a 0.1M phosphate buffer solution (pH 6.5) containing 5 mM EDTA, and the amount of hydroxyproline was measured by colorimetry on a microplate. The results were expressed by mean values (n = 4) of the percent liberation of hydroxyproline liberating from the fragment of articular cartilage into the culture medium ± standard deviation according to the following formula.

Percent Hydroxyproline Liberation = [Amount of Hydroxyproline in Supernatant of Culture/(Amount of Hydroxyproline in Supernatant of Culture + Amount of Hydroxyproline in Cartilage Residue)] x 100

As shown in Fig. 10, "conjugate 7" at a concentration of 1 mg/ml significantly inhibited the destruction of cartilage collagen induced by interleukin 1 and plasminogen but HA did not significantly inhibit the destruction.
These results show that the conjugate of HA and MMPI have a distinct inhibitory action on the destruction of the direct target tissue, anticular cartilage.
Further, all contents of the disclosure in the specifications of Japanese Patent Application Nos. Hei 10-138329/1998, Hei 10-224187/1998 and Hei 11-43064/1999 on the basis of which the present application priority claims are incorporated herein by reference.

INDUSTRIAL APPLICABILITY

The conjugate of the invention if administered into articular cavities is retained for as long a period as known HA formulations and inhibits localized MMP by the hydroxamic acids which are bound to HA or an HA derivative or a salt thereof. With the existing technology, it has been impossible to localize and prolong the action of therapeutics for joint disease (such as MMP inhibitor) at sites of administration (such as joints at the knee, shoulder and the like) and to reduce the frequency of their administration. These needs can be met by the conjugate of the invention which is expected to reduce the side effects of therapeutics for joint diseases considerably as compared to the conventional method of systemic administration.
At the site of administration, either HA or an HA derivative or a salt thereof which are the active ingredient of HA formulations or the therapeutic for joint disease exhibit their own efficacies to produce the desired synergism as they can show their activities without being dissociated or decomposed.
For these reasons, the conjugate of the invention features enhanced utility both as a therapeutic for joint disease (e.g. MMP inhibitor such as hydroxamic acid) and as HA or an HA derivative or a salt thereof and, hence, is useful as a drug with enhanced ability to suppress joint destruction; the conjugate is therefore anticipated to be an effective drug for treating osteoarthritis, rheumatoid arthritis or scapulohumeral periarthritis.

## Claims

1. A conjugate of (1) at least one therapeutic agent for joint diseases and (2) hyaluronic acid, a hyaluronic acid derivative or a salt thereof.

2. The conjugate of claim 1, wherein the bond between at least one therapeutic agent for joint diseases and hyaluronic acid, a hyaluronic acid derivative or a salt thereof is a covalent bond.

3. The conjugate of claim 1 or 2, wherein the therapeutic agent for joint diseases is a matrix metalloprotease inhibitor.

4. The conjugate of any one of claims 1 to 3, wherein the matrix metalloprotease inhibitor binds to hyaluronic acid, a hyaluronic acid derivative or the salt thereof via a spacer.

**5.** The conjugate of any one of claims 1 to 4, wherein the weight ratio of the matrix metalloprotease inhibitor to the entire conjugate is 0.01 to 50%.

**6.** The conjugate of any one of claims 1 to 5, wherein the matrix metalloprotease inhibitor is a hydroxamic acid residue.

**7.** The conjugate of any one of claims 1 to 6, wherein the matrix metalloprotease inhibitor is a hydroxamic acid residue represented by the general formula (1):

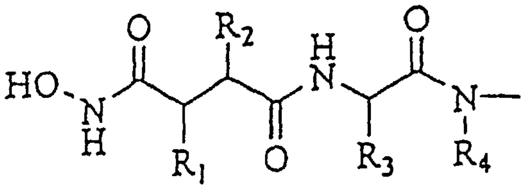

(1)

wherein

$R_1$ is a hydrogen atom, a hydroxyl group or a straight-chain or branched-chain alkyl group having 1 to 8 carbon atoms;
$R_2$ is a straight-chain or branched-chain alkyl group having 1 to 8 carbon atoms;
$R_3$ is a straight chain or branched alkyl group having 1 to 8 carbon atoms which may be substituted with a cycloalkyl group, an aryl group or a heterocyclic group; and
$R_4$ is a hydrogen atom or an alkyl group having 1 to 4 carbon atoms.

**8.** The conjugate of any one of claims 1 to 7, wherein the spacer is represented by the general formula (2):

$$-R_5-R_6-R_7-R_8- \quad (2)$$

wherein

$R_5$ is a straight-chain or branched-chain alkylene group having 1 to 8 carbon atoms;
$R_6$ is an oxygen atom or a methylene or imino group which may be substituted with a straight-chain or branched-chain alkyl group having 1 to 4 carbon atoms;
$R_7$ is a straight-chain or branched-chain alkylene group having 1 to 10 carbon atoms into which one to three oxygen atoms may be inserted; and
$R_8$ is an oxygen atom, a sulfur atom or $NR_9$ wherein $R_9$ is a hydrogen atom or a straight-chain or branched-chain alkyl group having 1 to 4 carbon atoms.

**9.** The conjugate of any one of claims 1 to 8, wherein the conjugate of the matrix metalloprotease inhibitor and the spacer is represented by the general formula (3):

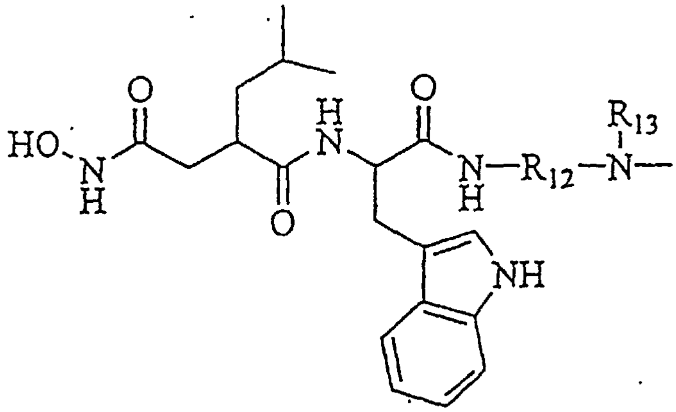

(3)

wherein

$R_{12}$ is a straight-chain or branched-chain alkylene group having 2 to 23 carbon atoms into which one imino group and/or one to four oxygen atoms may be inserted; and
$R_{13}$ is a hydrogen atom or a straight-chain or branched-chain alkyl group having 1 to 4 carbon atoms.

**10.** The conjugate of any one of claims 1 to 9, wherein the matrix metalloprotease inhibitor in the form of a conjugate with hyaluronic acid, a hyaluronic acid derivative or a salt thereof inhibits a matrix metalloprotease *in situ*.

**11.** A method for preparing the conjugate of any one of claims 1 to 10 comprising binding a site of the therapeutic agent for joint diseases that does not affect the activity of the agent to a carboxyl group, a hydroxyl group or a functional group at the reducing end of hyaluronic acid, a hyaluronic acid derivative or a salt thereof by direct chemical reaction or via a spacer.

**12.** A pharmaceutical composition comprising the conjugate of any one of claims 1 to 10.

**13.** The pharmaceutical composition of claim 12 which is a therapeutic agent for joint disease.

**14.** The pharmaceutical composition of claim 13, wherein the joint disease is osteoarthritis, rheumatoid arthritis or scapulohumeral periarthritis.

**15.** The use of the conjugate of any one of claims 1 to 10 in the preparation of a pharmaceutical composition.

**16.** The use of the conjugate of any one of claims 1 to 10 in the preparation of a therapeutic agent for joint diseases.

**17.** A method for treating a patient having a joint disease comprising administering a pharmaceutical composition containing a pharmaceutically effective amount of the conjugate of any one of claims 1 to 10 as the effective ingredient to the patient.

Fig. 1

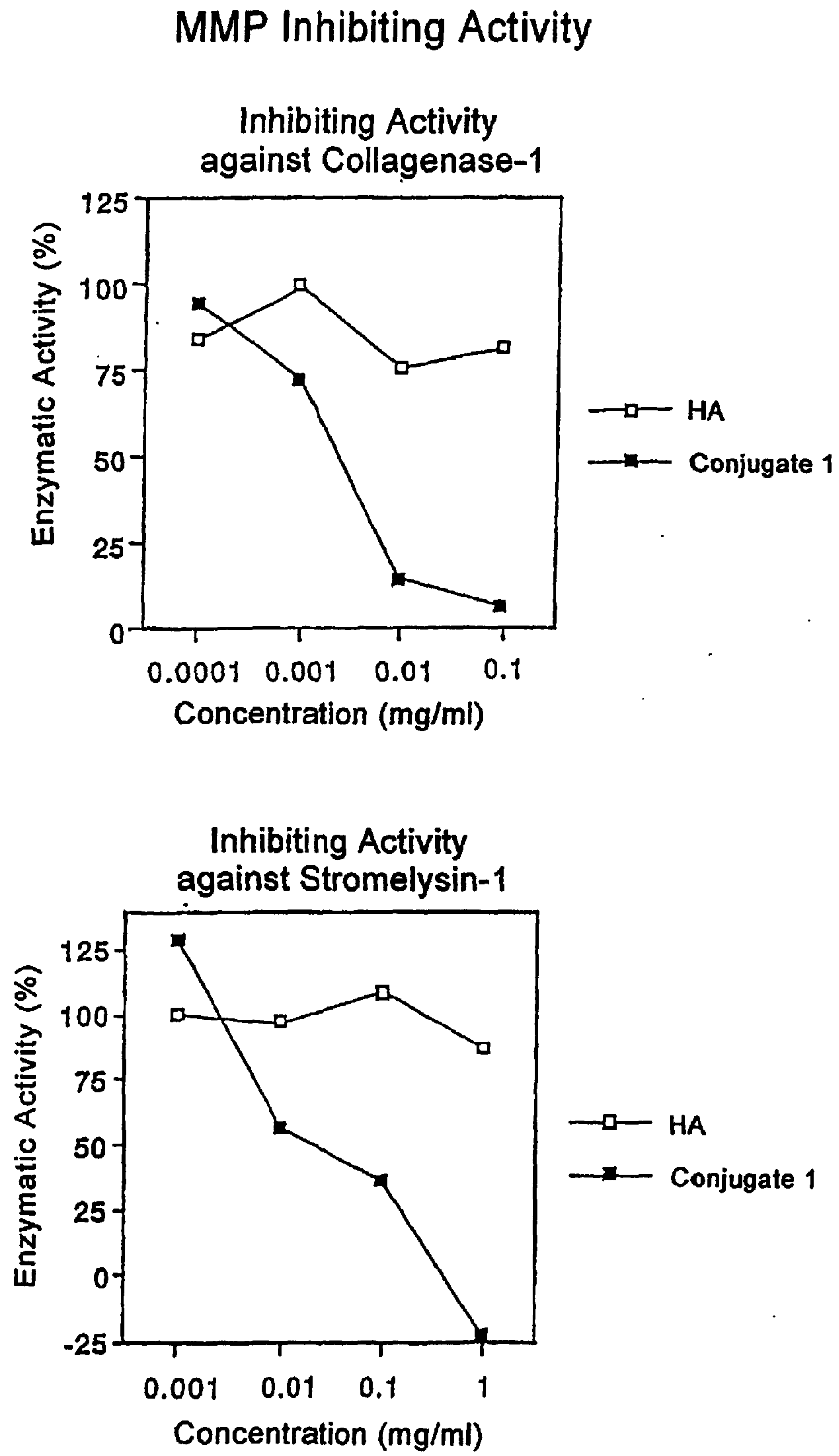
MMP Inhibiting Activity
Inhibiting Activity
against Collagenase-1
Enzymatic Activity (%)
125
100
75
50
25
0
0.0001
0.001
0.01
0.1
Concentration (mg/ml)
HA
Conjugate 1
Inhibiting Activity
against Stromelysin-1
Enzymatic Activity (%)
125
100
75
50
25
0
-25
0.001
0.01
0.1
1
Concentration (mg/ml)
HA
Conjugate 1

*Fig. 2*

MMP Inhibiting Activity

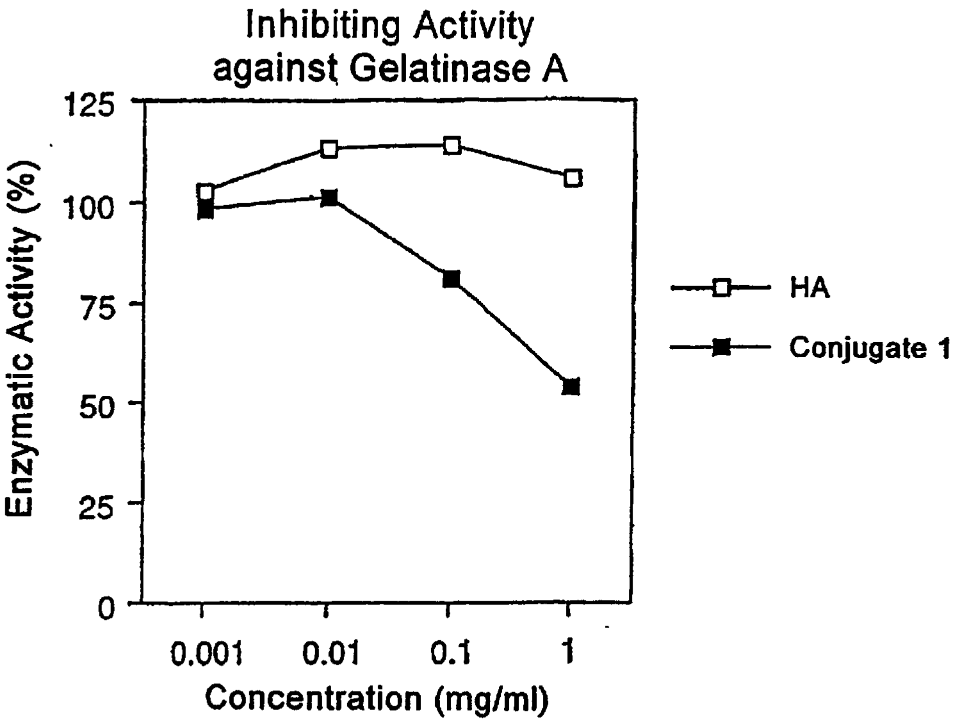


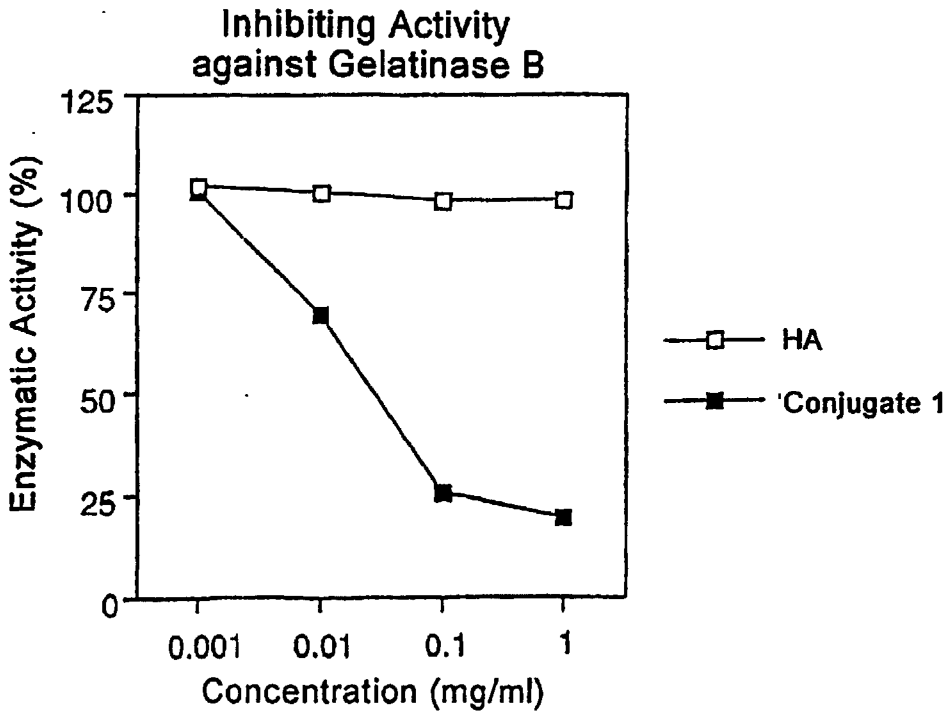

Fig. 3

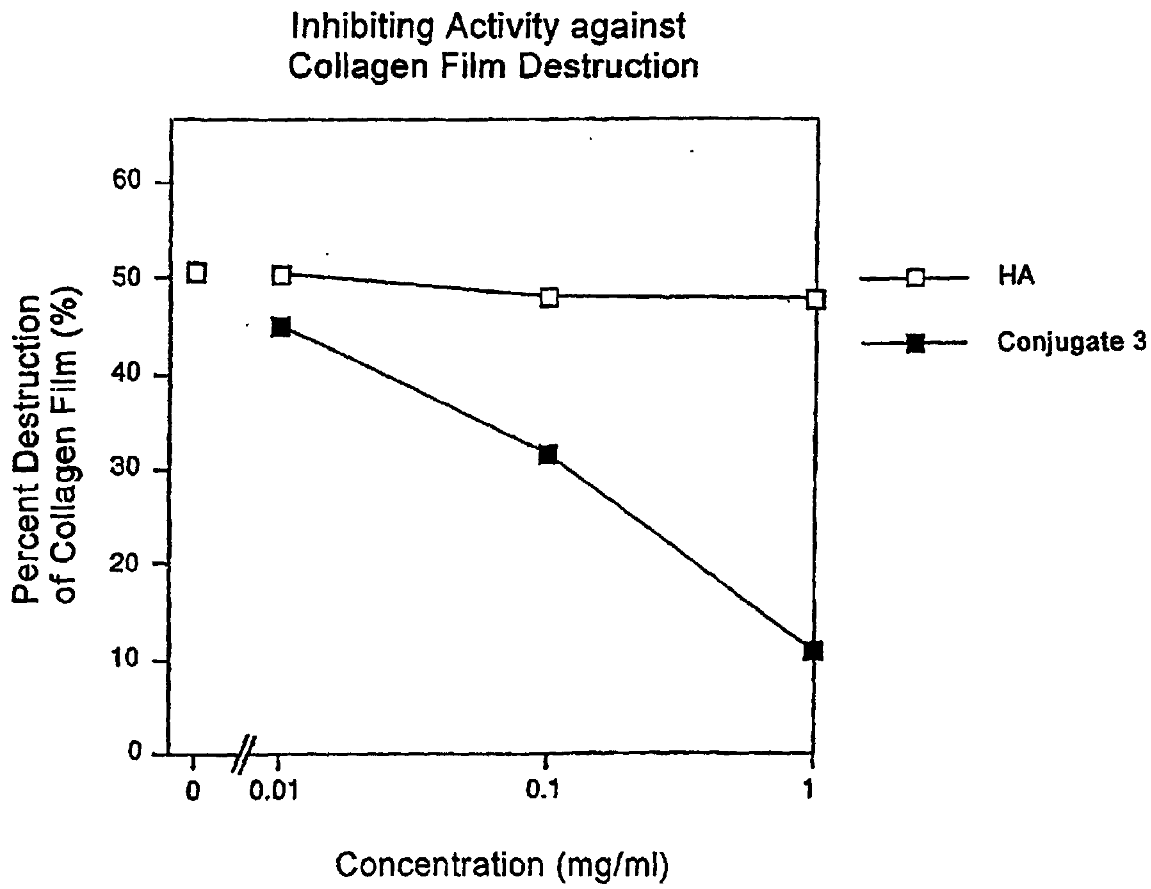
Inhibiting Activity against
Collagen Film Destruction
Percent Destruction
of Collagen Film (%)
60
50
40
30
20
10
0
0
0.01
0.1
1
Concentration (mg/ml)
HA
Conjugate 3

*Fig. 4*

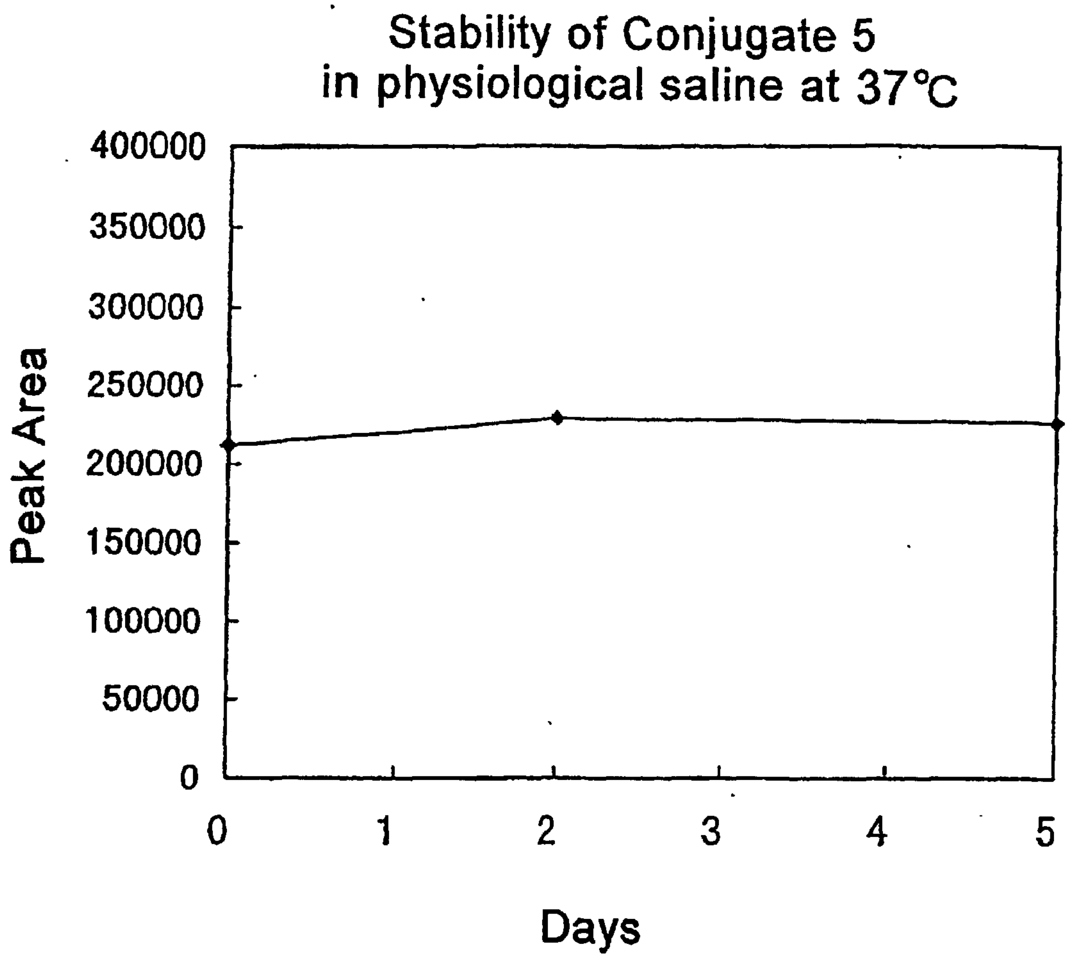
Stability of Conjugate 5
in physiological saline at 37°C
400000
350000
300000
250000
200000
150000
100000
50000
0
Peak Area
0
1
2
3
4
5
Days

Fig. 5

Permeability of Conjugate 4 against Semipermeable Membrane

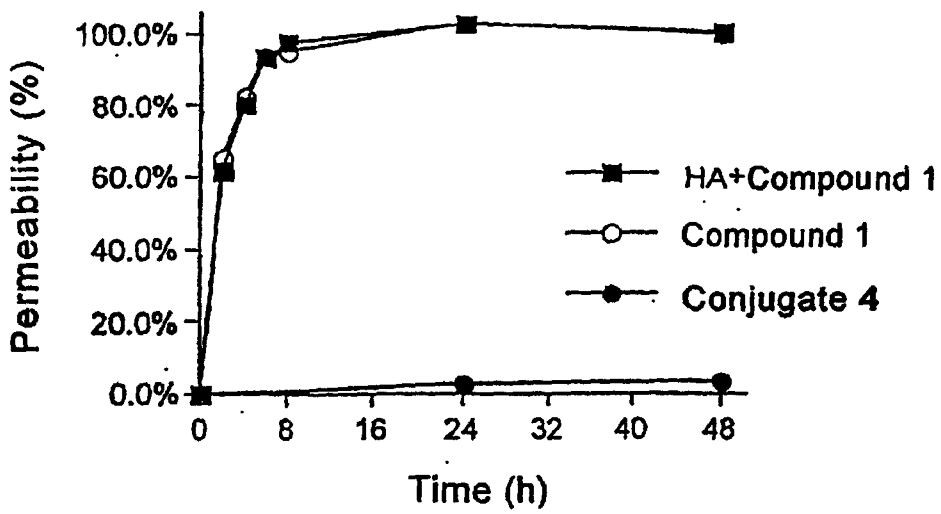

Fig. 6

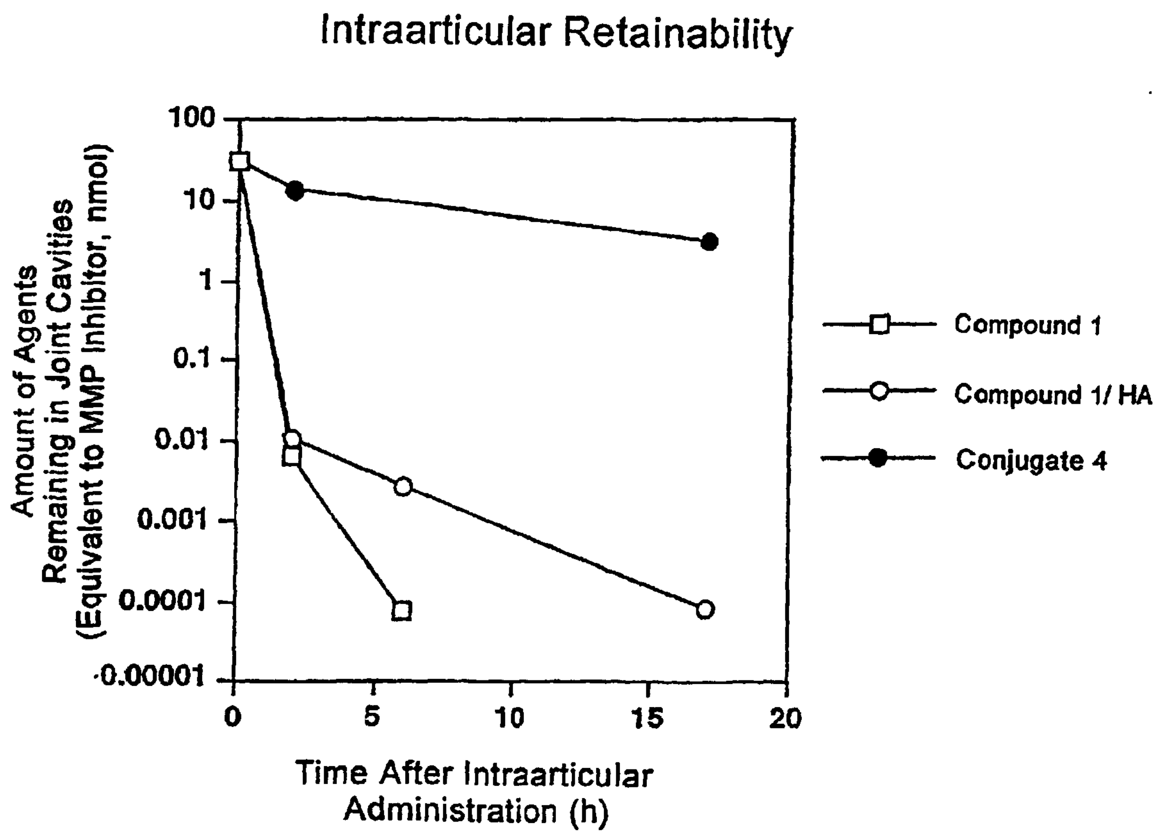
Intraarticular Retainability
Amount of Agents
Remaining in Joint Cavities
(Equivalent to MMP Inhibitor, nmol)
100
10
1
0.1
0.01
0.001
0.0001
0.00001
0
5
10
15
20
Time After Intraarticular
Administration (h)
Compound 1
Compound 1/ HA
Conjugate 4

Fig. 7

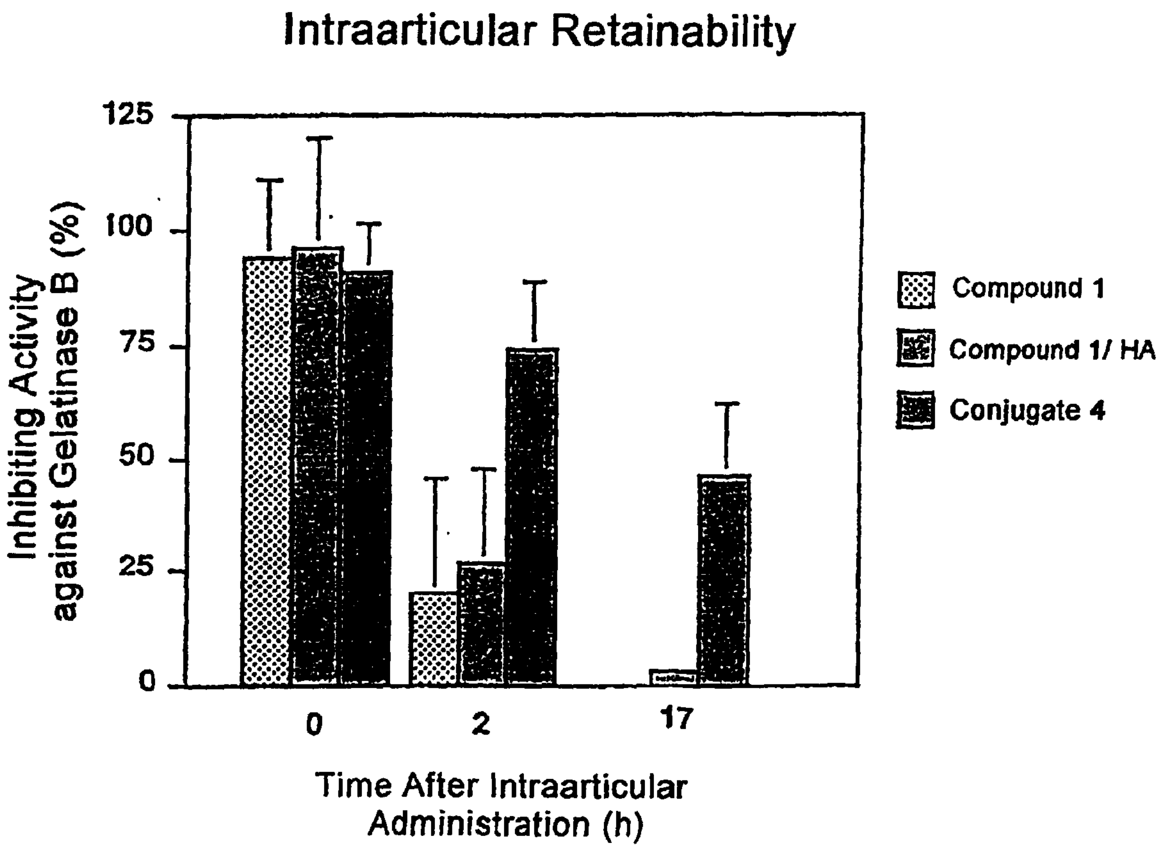
Intraarticular Retainability
Inhibiting Activity against Gelatinase B (%)
125
100
75
50
25
0
0
2
17
Time After Intraarticular Administration (h)
Compound 1
Compound 1/ HA
Conjugate 4

Fig. 8

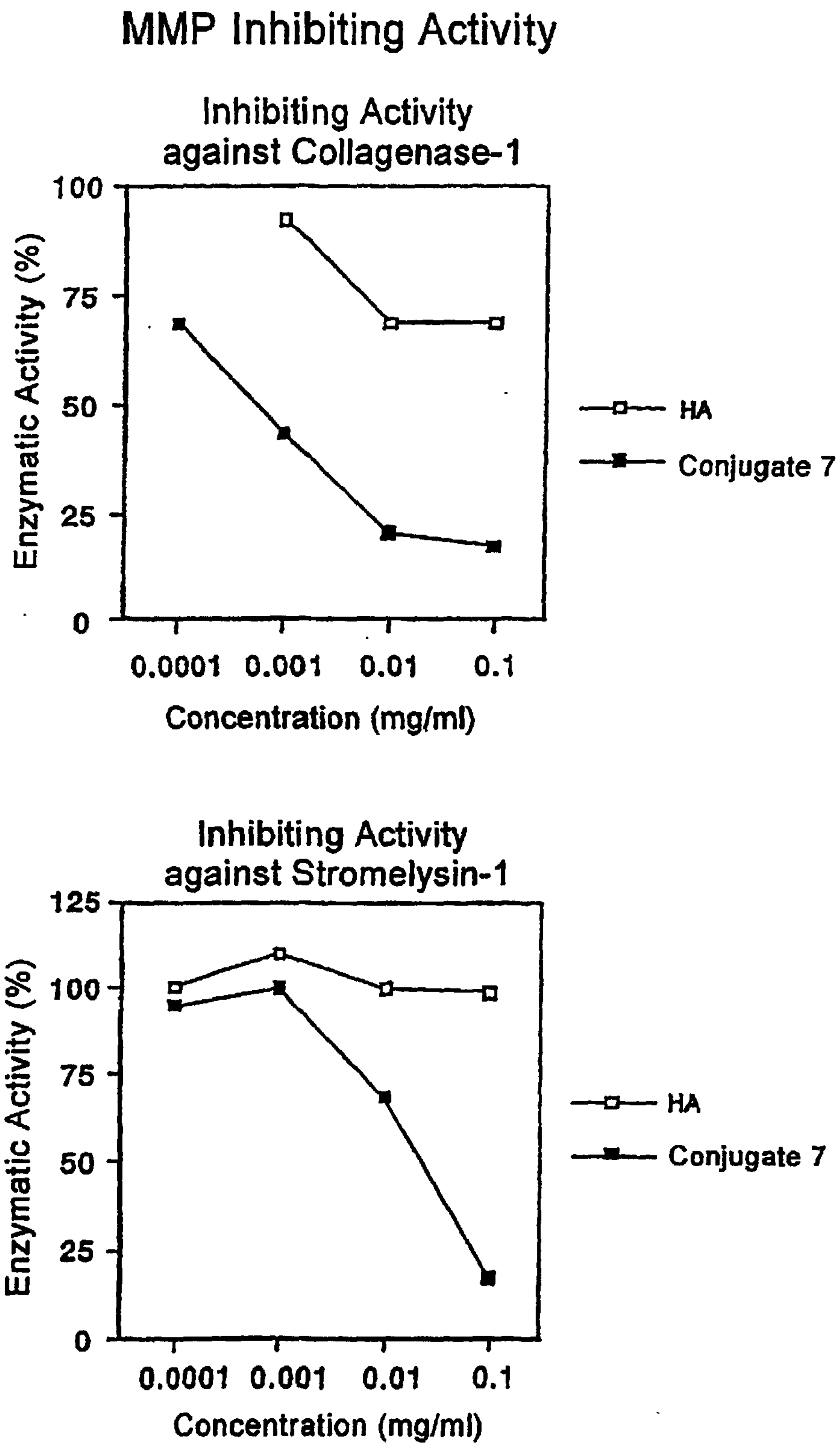
MMP Inhibiting Activity
Inhibiting Activity
against Collagenase-1
Enzymatic Activity (%)
100
75
50
25
0
0.0001 0.001 0.01 0.1
Concentration (mg/ml)
HA
Conjugate 7
Inhibiting Activity
against Stromelysin-1
Enzymatic Activity (%)
125
100
75
50
25
0
0.0001 0.001 0.01 0.1
Concentration (mg/ml)
HA
Conjugate 7

Fig. 9

MMP Inhibiting Activity

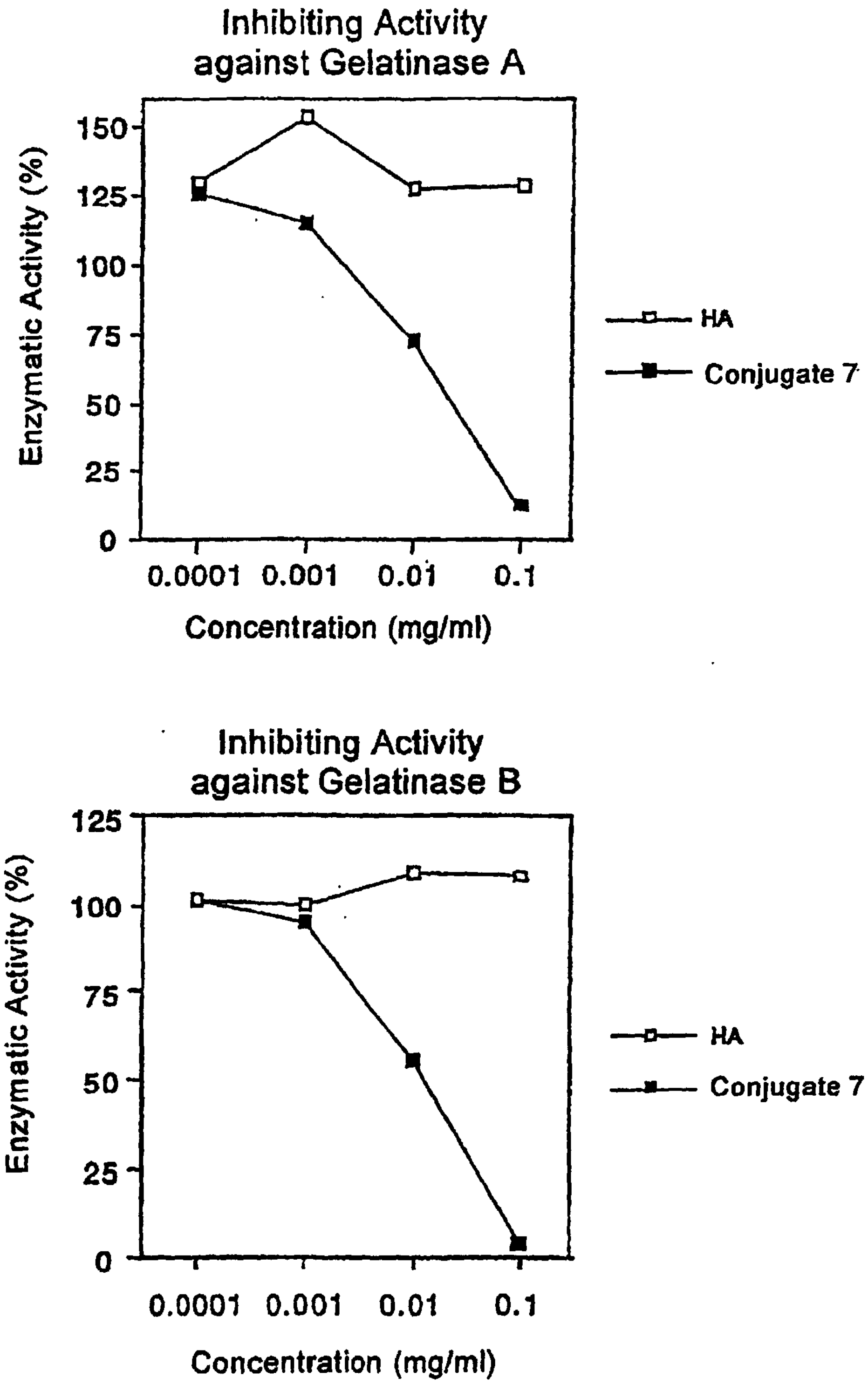

# Fig. 10

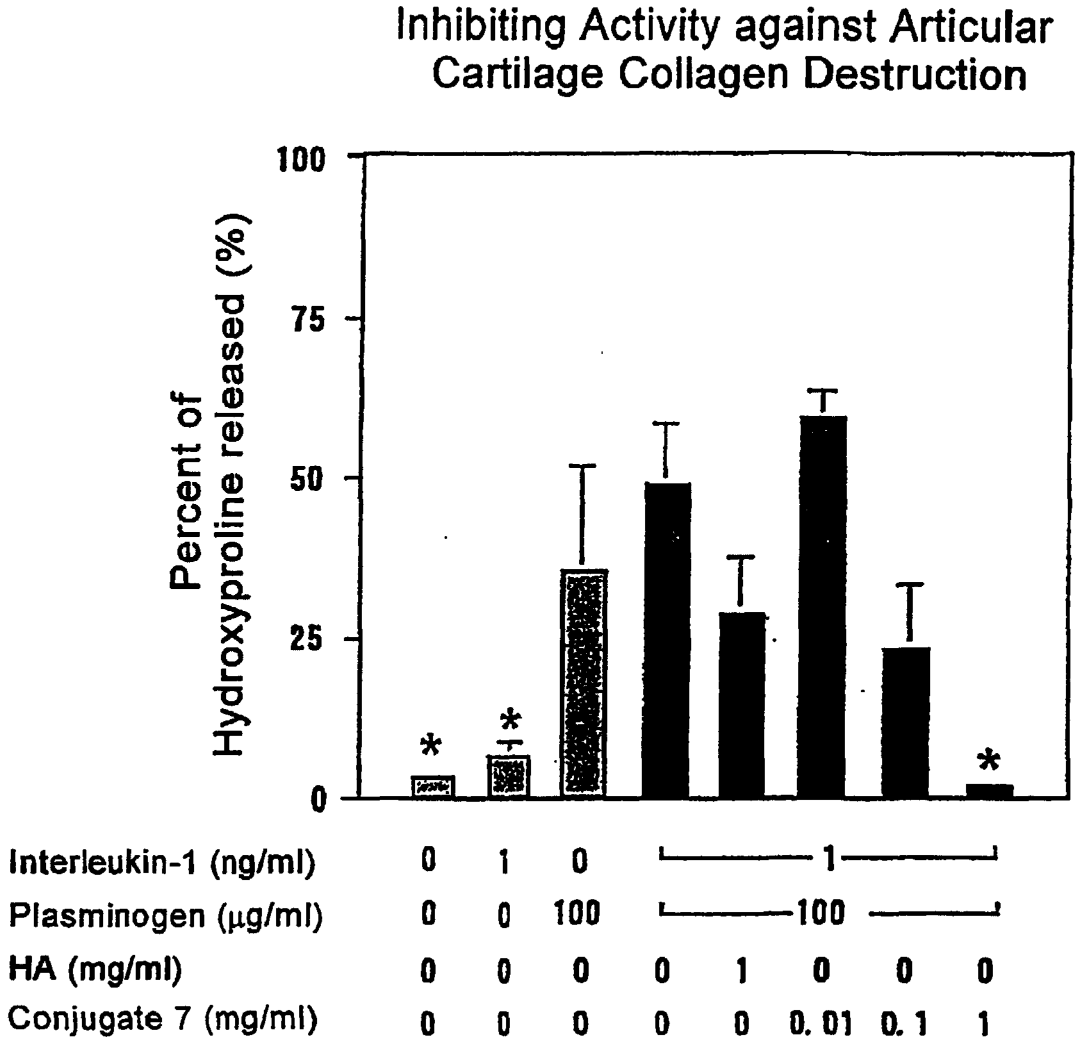
Inhibiting Activity against Articular Cartilage Collagen Destruction
Percent of Hydroxyproline released (%)
100
75
50
25
0
*
*
*
Interleukin-1 (ng/ml) 0 1 0 1
Plasminogen (μg/ml) 0 0 100 100
HA (mg/ml) 0 0 0 0 1 0 0 0
Conjugate 7 (mg/ml) 0 0 0 0 0 0.01 0.1 1

## INTERNATIONAL SEARCH REPORT

International application No.
PCT/JP99/02600

A. CLASSIFICATION OF SUBJECT MATTER
Int.Cl$^6$ A61K31/725, C08B37/08, A61K45/00 // (A61K31/725, 31:40)

According to International Patent Classification (IPC) or to both national classification and IPC

B. FIELDS SEARCHED

Minimum documentation searched (classification system followed by classification symbols)
Int.Cl$^6$ A61K31/725, C08B37/08, A61K45/00 // (A61K31/725, 31:40)

Documentation searched other than minimum documentation to the extent that such documents are included in the fields searched

Electronic data base consulted during the international search (name of data base and, where practicable, search terms used)
CAPLUS (STN), REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP, 62-64802, A (Fidia S.p.A.), 23 March, 1987 (23. 03. 87), Claims ; page 10, upper right column, line 11 to page 11, upper right column, line 20 ; page 18, upper left column, line 19 to page 20, lower left column, line 16 ; Examples 10 to 21 & EP, 216453, A & US, 4851521, A & US, 4965353, A & US, 5202431, A & US, 5336767, A | 1, 2, 12-16<br>3-11 |
| X<br>Y | Vasilionkaitis, V. Search for an artificial lubricant for joints based on complexes of poly(vinyl chloride) with hyaluronic acid biopolymers, Sint. Izuch. Fiziol. Akt. Veshchestv, Tezisy Dokl. Mezhvuz Nauchn. Konf. Uchastiem Farmakol. Latv. Est. SSR, 1975, 20-1 Publisher: Vil'nyus. Gos. Univ., Vilnius, USSR. Reference as a whole & Chem. abstr., Vol. 85, 1976 (Columbus, OH, USA), the abstract No. 99131 | 1, 2, 12-16<br>3-11 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 July, 1999 (22. 07. 99) | 3 August, 1999 (03. 08. 99) |
| Name and mailing address of the ISA/<br>Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP99/02600

C (Continuation). DOCUMENTS CONSIDERED TO BE RELEVANT

| Category* | Citation of document, with indication, where appropriate, of the relevant passages | Relevant to claim No. |
|---|---|---|
| Y | JP, 9-501183, A (Glycomed Inc.), 4 February, 1997 (04. 02. 97), Reference as a whole & WO, 95/199965, A1 & EP, 690841, A & US, 5773438, A & US, 5892112, A | 3-11 |

Form PCT/ISA/210 (continuation of second sheet) (July 1992)

## INTERNATIONAL SEARCH REPORT

International application No.
PCT/JP99/02600

**Box I Observations where certain claims were found unsearchable (Continuation of item 1 of first sheet)**

This international search report has not been established in respect of certain claims under Article 17(2)(a) for the following reasons:

1. [X] Claims Nos.: 17
   because they relate to subject matter not required to be searched by this Authority, namely:
   Claim 17 pertains to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.
2. [ ] Claims Nos.:
   because they relate to parts of the international application that do not comply with the prescribed requirements to such an extent that no meaningful international search can be carried out, specifically:
3. [ ] Claims Nos.:
   because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:
4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1992)